# EUROPEAN PATENT APPLICATION

(11) **EP 4 789 684 A1**
(43) Date of publication of application: **12.08.2026**
(21) Application number: 25382083.1
(22) Date of filing: 05.02.2025
(51) Int. Cl.: A61K 47/68, A61P 35/00

(54) **ANTI-ENDOGLIN ANTIBODY-DRUG CONJUGATES**

(71) Applicant: Oncomatryx Biopharma, S.L., 48160 Derio (Vizcaya) (ES)
(72) Inventor: The designation of the inventor has not yet been filed
(74) Representative: Mewburn Ellis LLP

(57) **Abstract**

The present invention relates to an antibody-drug conjugate having a structure of Formula (I), or a pharmaceutically acceptable salt or solvate thereof: 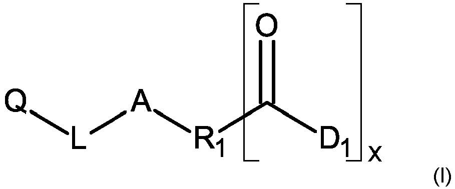 wherein Q is an antibody that selectively binds Endoglin; L is a linker; A is a bridging group; R₁ is a spacer; and D₁ is MMAE.

## Description

### Field of the Invention

The present invention relates to antibody-drug conjugates (ADCs) that target Endoglin (ENG), and to their use in a method of treating a disease, e.g. in the treatment of cancer.

### Background

Antibody-drug conjugates (ADCs) are a rapidly growing class of biopharmaceutical drugs intended as directed therapies for the treatment of cancer. These are highly targeted molecules that combine the specificity of surface antigen-specific monoclonal antibodies (mAbs) with the highly potent cytotoxicity of effector small chemical molecules. Their purpose is to induce target cell death, thereby attacking and killing tumor cells while sparing healthy cells. The primary mechanism of action of ADCs involves the specific binding of the monoclonal antibody component to target antigens on the surface of cancer cells. Upon binding, the ADC is internalized by cancer cells, resulting in intracellular release of cytotoxic payload and ultimately cell death.

ADCs have emerged as a powerful class of therapeutic drugs and show promising effect in the treatment of various cancer types. They have the potential to improve therapeutic efficacy and reduce systemic toxicity that often occurs with small molecule drugs. They are designed to maximize antitumor activity while minimizing damage on normal tissue, potentially improving the therapeutic index. A variety of ADCs have been approved by the FDA, and nearly 100 ADCs are currently under development, undergoing preclinical and clinical studies (Shastry, Mythili *et al.*; Fu, Zhiwen *et al.*; Pettinato MC).

Recent studies suggest that therapeutic agents designed to inhibit TGF-β signaling pathway at the tumour-stroma interphase could prevent cancer progression, improving prognosis and treatment. The TGF-β co-receptor family has emerged as a target for cancer treatments acting on the tumour or on its neovasculature. Endoglin (ENG, CD105), an accessory protein of the type II TGF-β receptor complex, is part of this family.

WO 2015/118031 discloses an anti-ENG antibody-drug conjugate, which may otherwise be referred to as OMTX703, wherein the drug comprises a cytolysin or a Nigrin-b A-chain, and *in vivo* anti-tumour activity of this ADC.

Cytolysins are a class of synthetic tetrapeptide analogs of natural tubulysins, which have low molecular weight and show high cytotoxic activity. They are not substrates of Pgp and thus do not generate multidrug resistance. They exert their mechanism of action by binding to tubulin to inhibit its polymerization and prevent microtubule depolymerization. This results in the disruption of microtubules, leading to disassembly and cell cycle arrest. Therefore, cytolysins are used as payloads of ADCs, such as OMTX705, due to their potent cytotoxic effects.

There are many possible payloads for ADCs. These can generally be categorised based on their mechanism of action, for example tubulin inhibitors, DNA damaging agents and immunomodulators (see e.g. Fu et al. Nature (2022) 7(1):93). When it comes to ADC design, there are many options for targets, payloads and linkers, and each component of the ADC must be compatible to achieve high specificity, efficacy and safety (see *e.g*. Marei et al. Cancer Cell International (2022) 22(1):255). One example of a cytotoxic payload used in ADCs is the dolastatin 10 derivative, monomethyl auristatin E.

Monomethyl auristatin E (MMAE) is another potent cytotoxic agent commonly used as payload of antibody-drug conjugates (ADCs). It inhibits cell division by blocking tubulin polymerization and exerts cytotoxic effects by binding to microtubules and preventing cell proliferation. MMAE meets key requirements for an ideal ADC payload, including high cytotoxicity, well-defined target and mechanism of action, potential chemical binding sites, high stability during circulation, uptake and release into the target, and good solubility. Studies have shown that MMAE is a substrate of multiple drug resistance and is therefore secreted by MDR1 and excreted from cells. This MDR1-mediated efflux mechanism may influence the cytotoxic effects of antibody-drug conjugates (ADCs) containing MMAE payloads. This payload is used in FDA-approved ADCs such as Adcetris^{®} and Polivy^{®} and has shown potent activity in clinical trials for various cancers, including lymphoma, leukemia, lung, gastric, prostate, and breast cancer.

While both MMAE and cytolysin are used as payloads in ADCs for cancer therapy, they differ in their mechanisms of action, interactions with drug resistance proteins, and potential implications for toxicity and safety of the corresponding ADCs (Wang, Zhijia *et al.*) (Samantasinghar, Anupama *et al.*) (Riccardi, Federico *et al.*)*.*

The present invention has been devised in light of the above considerations.

### Summary of the Invention

Broadly, the present invention relates to antibody-drug conjugates, wherein the antibody selectively binds endoglin (ENG) and is conjugated to a drug comprising MMAE. The present inventors have found that anti-ENG antibody-drug conjugates as described herein exhibit highly specific binding and improved *in vitro* and *in vivo* cytotoxicity compared to other anti-ENG antibody-drug conjugates.

Accordingly, in a first aspect the present invention provides an antibody-drug conjugate having a structure of Formula (I), or a pharmaceutically acceptable salt or solvate thereof: 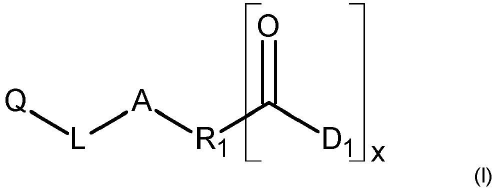 wherein:
**Q** is an antibody that selectively binds Endoglin;
L is a linker;
A is a bond or 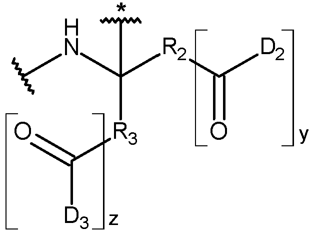 where the asterisk (*) marks the point of attachment to R₁;
each of D₁, D₂ and D₃ are independently MMAE;
each of x, y and z is independently 0 or 1, with a provision that at least one of x, y, and z is 1;
each of R₁, R₂, and R₃ is independently selected from: a bond, hydrogen, 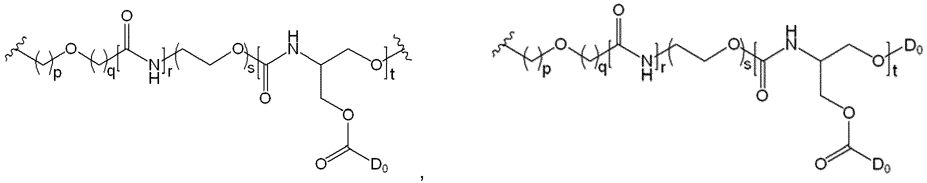 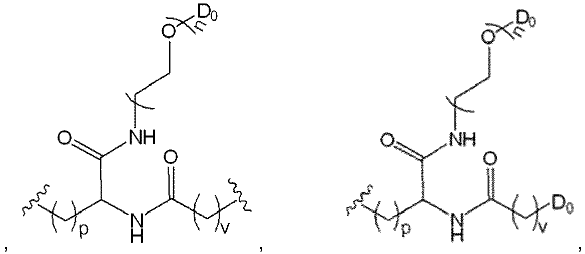 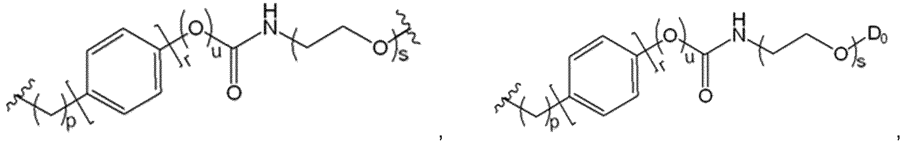 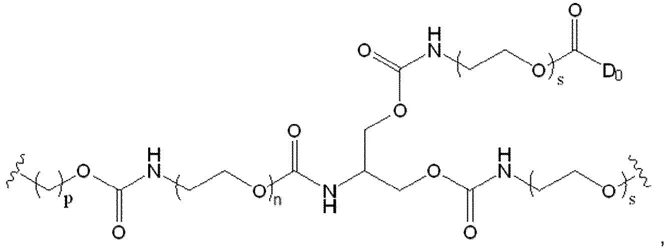 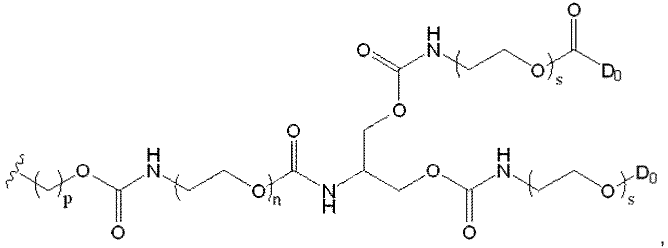 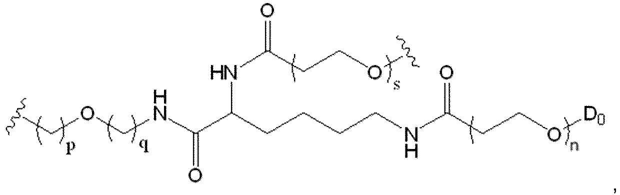 and 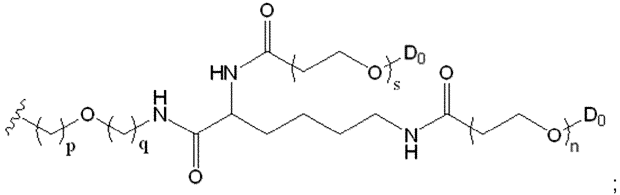
wherein:
   p is 1, 2, or 3;
   q is 0, 1, or 2;
   r is 0 or 1;
   s is an integer from 0 to 37;
   t is 0 or 1;
   u is 0 or 1;
   v is 1, 2, or 3; and
   n is an integer from 1 to 37; or
   x and y are 0, and R₁ and R₂ together form =O and R₃ is 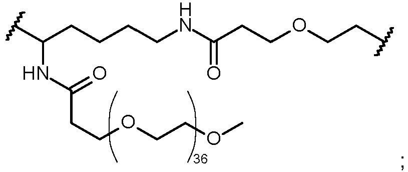
   D₀ is methyl, propargyl, or MMAE;
   with a provision that at least one of R₁, R₂, and R₃ is not hydrogen;
   if q, r, s, and t are 0, then x + y + z > 1;
   if x is 0, then R₁ is selected from: hydrogen, 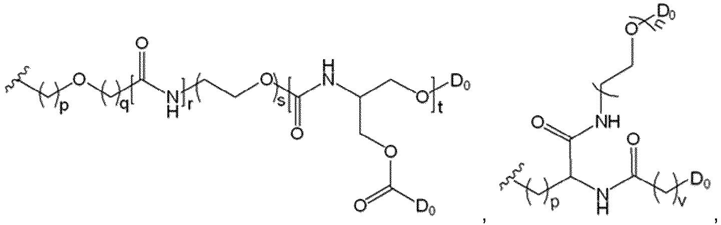 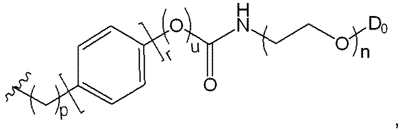 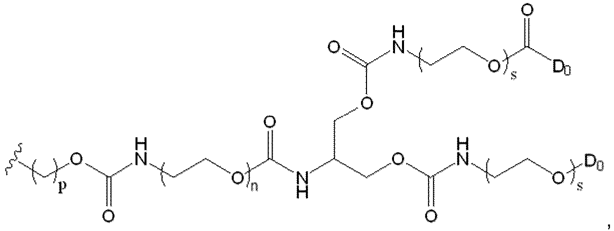 and 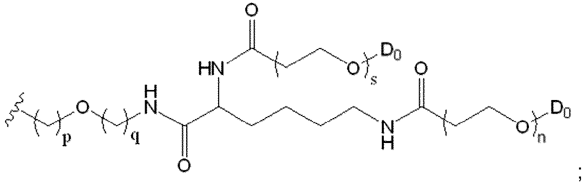
   if y is 0, then R₂ is selected from: hydrogen, 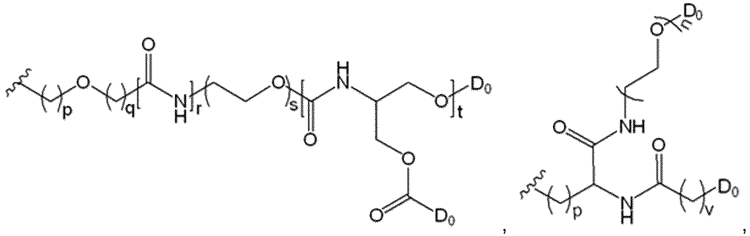 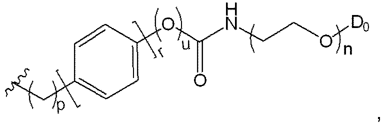 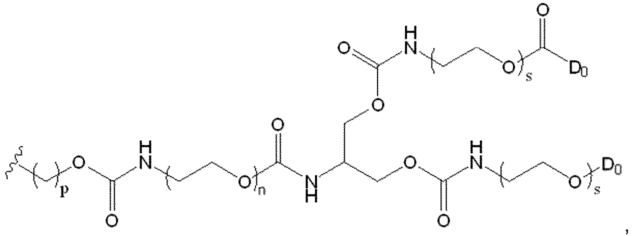 and 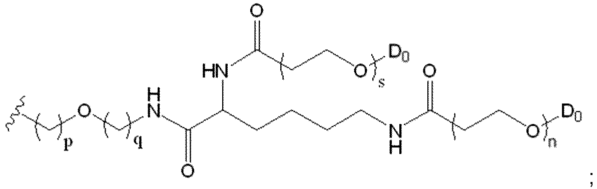
   if z is 0, then R₃ is selected from: hydrogen, 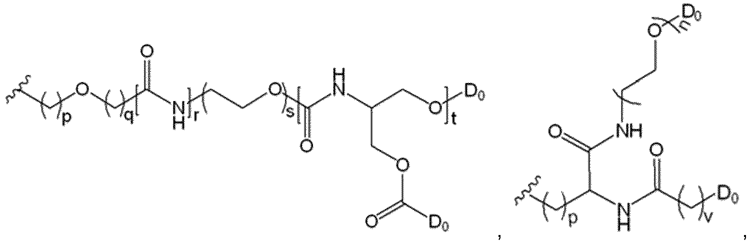 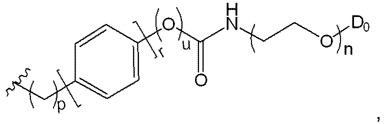 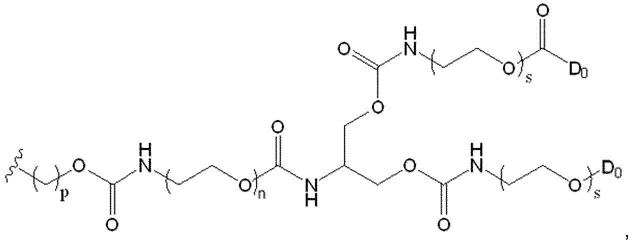 and 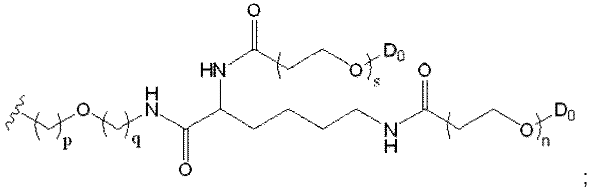
   if x + y + z = 1, and each of R₁, R₂, and R₃ independently is 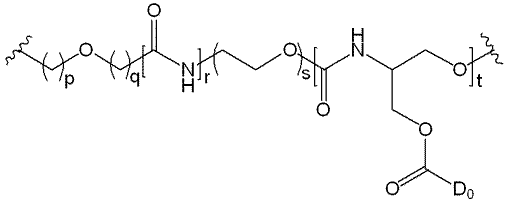 or 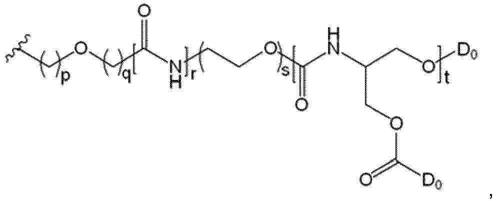 then p is not 1;
   if each of R₁, R₂, and R₃ independently is 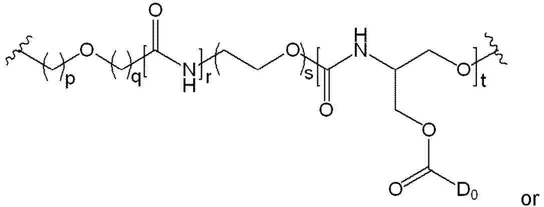 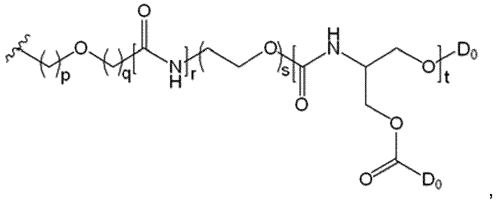 and r, s, and t are each 0, then x + y + z > 1.

In some embodiments, the conjugate has a structure according to Formula (II), or a pharmaceutically acceptable salt of solvate thereof: 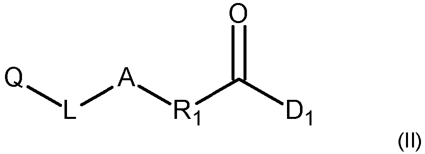 wherein:
Q is an antibody that selectively binds Endoglin;
L is a linker;
A is a bond or -NH-CH₂-;
D₁ is MMAE;
R₁ is selected from: a bond, 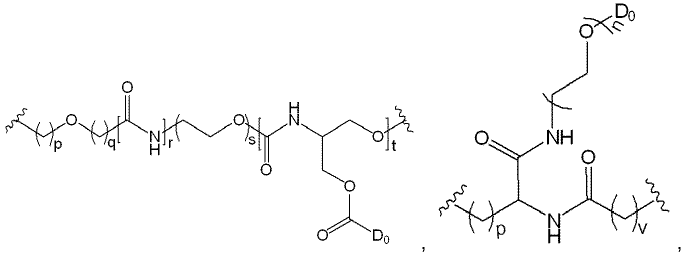 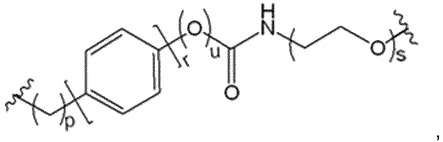 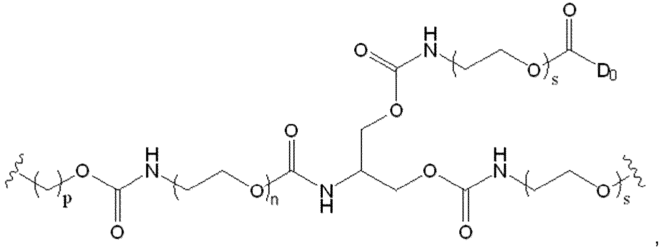 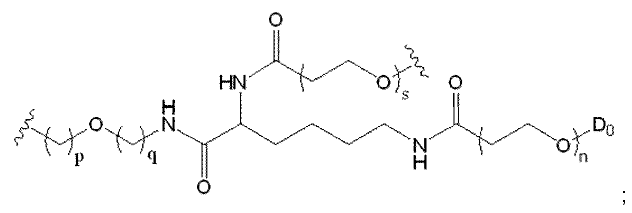
wherein:
   p is 1, 2, or 3;
   q is 0, 1, or 2;
   r is 0 or 1;
   s is an integer from 0 to 37;
   t is 0 or 1;
   u is 0 or 1;
   v is 1, 2, or 3; and
   n is an integer from 1 to 37; and
   if R₁ is 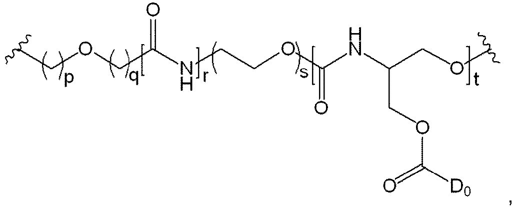 then p is not 1.

In some embodiments, L is selected from: 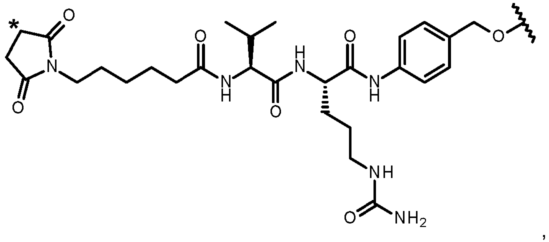 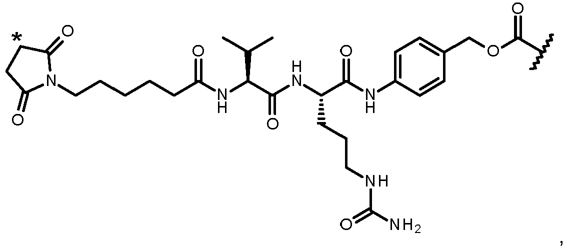 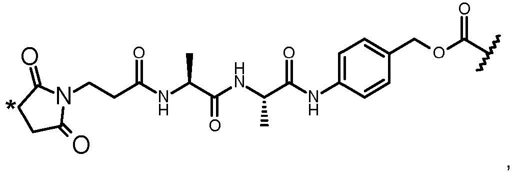 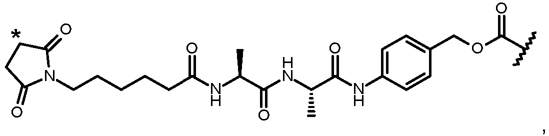 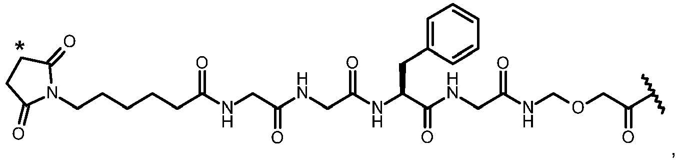 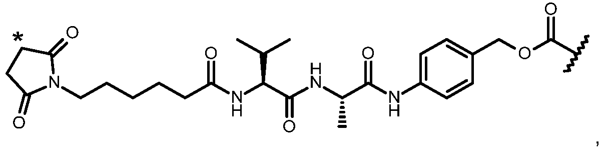 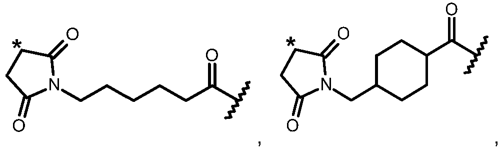 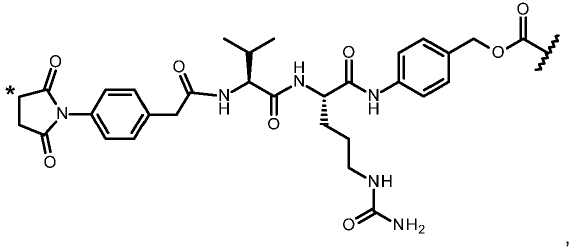 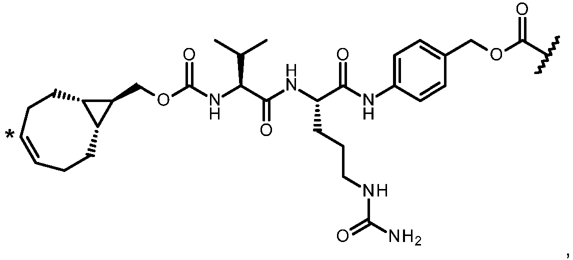 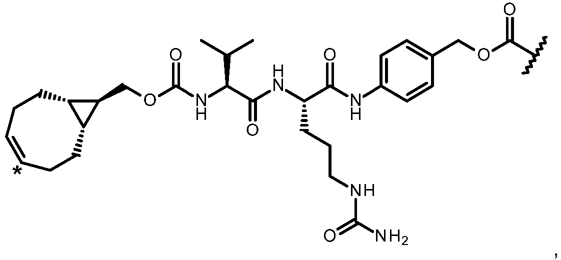 and 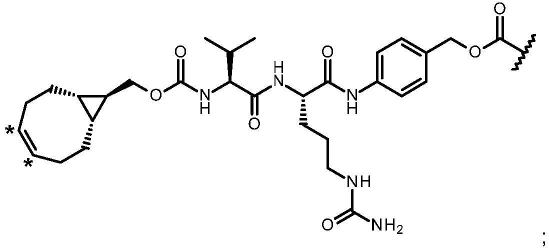 where the asterisk (*) marks the point of attachment to the antibody Q.

In some embodiments, L is: 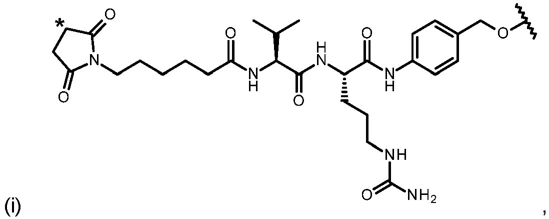 or 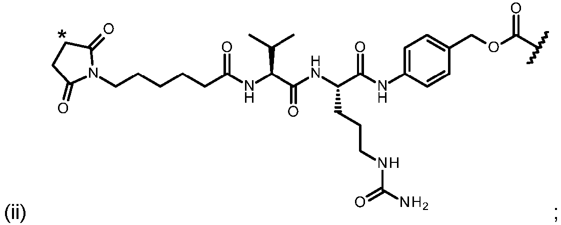 where the asterisk (*) marks the point of attachment to Q.

In some embodiments, R₁ is a bond or

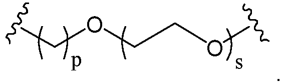

In some embodiments:
L has the structure 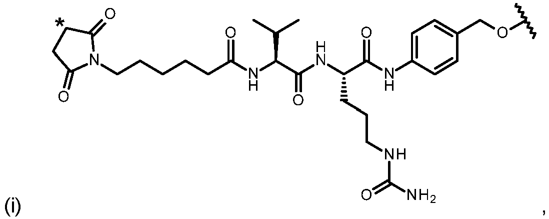
where the asterisk (*) marks the point of attachment to Q;
A is a bond; and
R₁ is a bond.

In some embodiments:
L has the structure 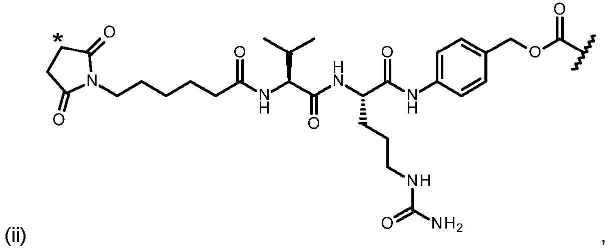
where the asterisk (*) marks the point of attachment to Q;
A is -NH-CH₂-; and
R₁ is

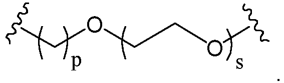

In some embodiments, p is 1. In some embodiments, s is 3. In some embodiments, p is 1 and s is 3.

In some embodiments, the conjugate is: 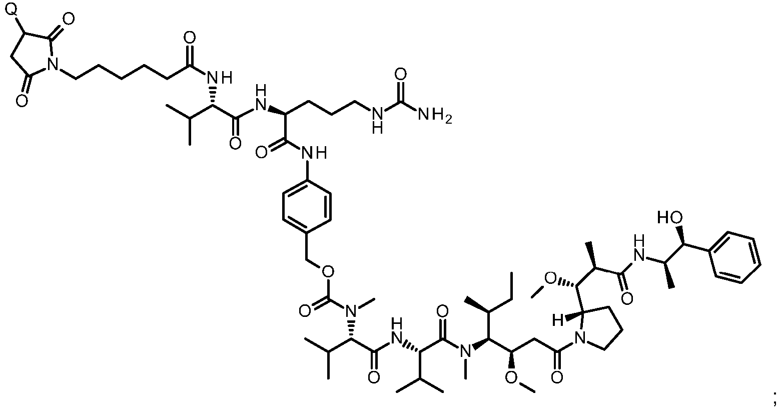 or 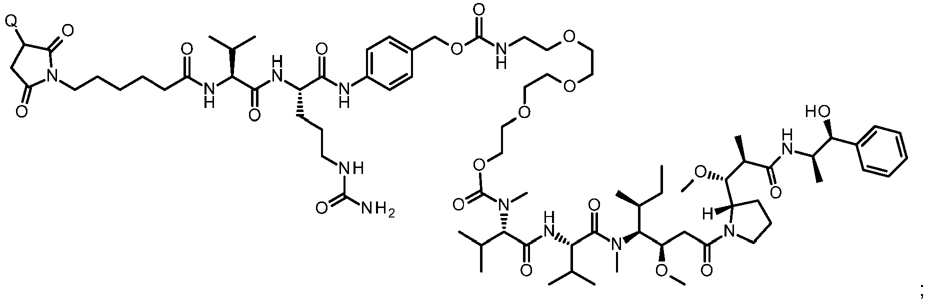 or a pharmaceutically acceptable salt or solvate thereof.

In some embodiments, the drug antibody ratio (DAR) is from about 3 to about 13. In some embodiments, the DAR is from about 3 to about 10. In some embodiments, the DAR is from about 3 to about 5. In some embodiments, the DAR is from about 7 to about 9.

In some embodiments, the antibody is a monoclonal antibody or binding fragment thereof that selectively binds to an extracellular region of human Endoglin. In some embodiments, the antibody comprises heavy chain complementarity determining regions 1-3 (CDRH1-3) and light chain complementarity determining regions 1-3 (CDRL1-3) having the following amino acid sequences:
(i) CDRH1: SEQ ID NO: 7 or a variant thereof having up to 1 or 2 amino acid substitutions compared with the sequence of SEQ ID NO: 7;
(ii) CDRH2: SEQ ID NO: 8 or a variant thereof having up to 1 or 2 amino acid substitutions compared with the sequence of SEQ ID NO: 8;
(iii) CDRH3: SEQ ID NO: 9 or a variant thereof having up to 1 or 2 amino acid substitutions compared with the sequence of SEQ ID NO: 9;
(iv) CDRL1: SEQ ID NO: 10 or a variant thereof having up to 1 or 2 amino acid substitutions compared with the sequence of SEQ ID NO: 10;
(v) CDRL2: SEQ ID NO: 11 or a variant thereof having up to 1 or 2 amino acid substitutions compared with the sequence of SEQ ID NO: 11; and
(vi) CDRL3: SEQ ID NO: 12 or a variant thereof having up to 1 or 2 amino acid substitutions compared with the sequence of SEQ ID NO: 12.

In some embodiments, CDRH1-3 comprise the amino acid sequences of SEQ ID NOS: 7-9, respectively and CDRL1-3 comprise the amino acid sequences of SEQ ID NOS: 10-12, respectively.

In some embodiments, the antibody comprises a heavy chain variable region (VH) comprising an amino acid sequence having at least 90%, 95% or 99% sequence identity with the full-length sequence of SEQ ID NO: 5.

In some embodiments, the antibody comprises a heavy chain variable region (VH) comprising the amino acid sequence of SEQ ID NO: 5.

In some embodiments, the antibody comprises a light chain variable region (VL) comprising an amino acid sequence having at least 90%, 95% or 99% sequence identity with the full-length sequence of SEQ ID NO: 6. In particular, the antibody may comprise a light chain variable region (VL) comprising the amino acid sequence of SEQ ID NO: 6.

In some embodiments, the antibody comprises a heavy chain comprising an amino acid sequence having at least 90%, 95% or 99% sequence identity with the full-length sequence of SEQ ID NO: 3. In particular, the antibody may comprise a heavy chain comprising the amino acid sequence of SEQ ID NO: 3.

In some embodiments, the antibody comprises a light chain comprising an amino acid sequence having at least 90%, 95% or 99% sequence identity with the full-length sequence of SEQ ID NO: 4. In particular, the antibody may comprise a light chain comprising the amino acid sequence of SEQ ID NO: 4.

In some embodiments, the antibody is a monoclonal antibody or binding fragment thereof that selectively binds to an extracellular region of murine Endoglin. Conjugates that target murine Endoglin find particular use in pre-clinical testing, e.g., employing well-characterised murine models of various cancers. In particular, the antibody may comprise heavy chain complementarity determining regions 1-3 (CDRH1-3) and light chain complementarity determining regions 1-3 (CDRL1-3) having the following amino acid sequences:
(i) CDRH1: SEQ ID NO: 19 or a variant thereof having up to 1 or 2 amino acid substitutions compared with the sequence of SEQ ID NO: 19;
(ii) CDRH2: SEQ ID NO: 20 or a variant thereof having up to 1 or 2 amino acid substitutions compared with the sequence of SEQ ID NO: 20;
(iii) CDRH3: SEQ ID NO: 21 or a variant thereof having up to 1 or 2 amino acid substitutions compared with the sequence of SEQ ID NO: 21;
(iv) CDRL1: SEQ ID NO: 22 or a variant thereof having up to 1 or 2 amino acid substitutions compared with the sequence of SEQ ID NO: 22;
(v) CDRL2: SEQ ID NO: 23 or a variant thereof having up to 1 or 2 amino acid substitutions compared with the sequence of SEQ ID NO: 23; and
(vi) CDRL3: SEQ ID NO: 24 or a variant thereof having up to 1 or 2 amino acid substitutions compared with the sequence of SEQ ID NO: 24. For example, CDRH1-3 may comprise the amino acid sequences of SEQ ID NOS: 19-21, respectively and CDRL1-3 may comprise the amino acid sequences of SEQ ID NOS: 22-24, respectively.

In some embodiments, the antibody comprises a heavy chain variable region (VH) comprising an amino acid sequence having at least 90%, 95% or 99% sequence identity with the full-length sequence of SEQ ID NO: 17, e.g. Q may comprise a heavy chain variable region (VH) comprising the amino acid sequence of SEQ ID NO: 17.

In some embodiments, the antibody comprises a light chain variable region (VL) comprising an amino acid sequence having at least 90%, 95% or 99% sequence identity with the full-length sequence of SEQ ID NO: 18, e.g., the antibody may comprise a light chain variable region (VL) comprising the amino acid sequence of SEQ ID NO: 18.

In some embodiments, the antibody comprises: a) a heavy chain variable region (VH) comprising an amino acid sequence having at least 90%, 95% or 99% sequence identity with the full-length sequence of SEQ ID NO: 17; optionally wherein the antibody comprises a heavy chain variable region (VH) comprising the amino acid sequence of SEQ ID NO: 17; and/or b) a light chain variable region (VL) comprising an amino acid sequence having at least 90%, 95% or 99% sequence identity with the full-length sequence of SEQ ID NO: 18; optionally wherein the antibody comprises a light chain variable region (VL) comprising the amino acid sequence of SEQ ID NO: 18.

In some embodiments, the antibody comprises a heavy chain comprising an amino acid sequence having at least 90%, 95% or 99% or even 100% sequence identity with the full-length sequence of SEQ ID NO: 15.

In some embodiments, the antibody comprises a light chain comprising an amino acid sequence having at least 90%, 95% or 99% or even 100% sequence identity with the full-length sequence of SEQ ID NO: 16.

In some embodiments, the antibody comprises: a) a heavy chain comprising an amino acid sequence having at least 90%, 95% or 99% or even 100% sequence identity with the full-length sequence of SEQ ID NO: 15; and/or b) a light chain comprising an amino acid sequence having at least 90%, 95% or 99% or even 100% sequence identity with the full-length sequence of SEQ ID NO: 16.

In a second aspect the present invention provides a conjugate as defined in accordance with the first aspect of the invention for use in a method of treating a disease in a mammalian subject. In some embodiments, the disease is a cancer or an inflammatory disease. In some embodiments, the disease is a cancer, arthritis, or fibrosis. In some embodiments, the disease is a cancer or arthritis (e.g. rheumatoid arthritis). In some embodiments, the disease is a cancer or fibrosis. In some embodiments, the disease is arthritis (e.g. rheumatoid arthritis) or fibrosis.

According to a third aspect, the present invention provides a conjugate as defined in accordance with the first aspect of the invention for use in a method of treating cancer in a mammalian subject. The cancer may comprise a solid tumour. Alternatively, the cancer may comprise blood cancer. In particular, the cancer may comprise gastric cancer, pancreatic cancer, Ewing sarcoma, breast cancer, melanoma, lung cancer, head & neck cancer, ovarian cancer, bladder cancer or colon cancer.

In some embodiments the cancer is an Endoglin-positive cancer (including any of the above-described cancers) that expresses Endoglin on one or more cancer cells and/or on one or more cells forming part of the tumour microenvironment, in particular, one or more cells of the stroma. The cancer may be any stage. In particular, the cancer may be a metastatic cancer. In particular, the cancer may be gastric cancer that expresses Endoglin.

In a fourth aspect, the present invention provides a conjugate as defined in accordance with the first aspect of the invention for use in a method of treating an inflammatory disease in a mammalian subject. The inflammatory disease may be fibrotic (e.g., fibrosis, e.g., pulmonary fibrosis), autoimmune (e.g., rheumatoid arthritis), allergic (e.g., asthma), infectious (e.g., tuberculosis), rheumatic (e.g., ankylosing spondylitis), gastrointestinal (e.g., Crohn's disease), dermatological (e.g., psoriasis), neurological (e.g., multiple sclerosis), pulmonary (e.g., chronic obstructive pulmonary disease), cardiovascular (e.g., myocarditis), renal (e.g., glomerulonephritis), endocrine (e.g., Hashimoto's thyroiditis), or systemic (e.g., systemic lupus erythematosus). In some embodiments, the inflammatory disease is fibrosis. The fibrosis may be of the liver, lung, heart and/or colon. In some embodiments, the inflammatory disease is arthritis, for example rheumatoid arthritis. In some embodiments, the inflammatory disease is selected from: fibrosis, arthritis, psoriasis, systemic lupus erythematosus (SLE), inflammatory bowel diseases such as Crohn's disease and ulcerative colitis, asthma, atopic dermatitis, ankylosing spondylitis, multiple sclerosis, Hashimoto's thyroiditis, and sarcoidosis. In some embodiments, the fibrosis is pulmonary fibrosis (e.g., idiopathic pulmonary fibrosis), liver fibrosis, or a cancer-associated fibrosis.

In some embodiments of any of the aspects, the conjugate is for simultaneous, sequential or separate administration with one or more other antitumour drugs. The one or more other antitumour drugs may comprise a cytotoxic chemotherapeutic agent or an anti-angiogenic agent or an immunotherapeutic agent. In some embodiments the one or more other antitumour drugs comprise Gemcitabine, Nab-paclitaxel (Abraxane^{®}), bevacizumab, itraconazole, carboxyamidotriazole, an anti-PD-1 molecule (for example, nivolumab, tislelizumab, or pembrolizumab) or an anti-PD-L1 molecule.

In a further aspect the present invention provides a method of treating a disease in a mammalian subject, the method comprising administering a therapeutically effective amount of a conjugate as defined in accordance with the first aspect of the invention to the subject in need thereof. The disease may comprise cancer. The cancer may be as defined above in relation to the third aspect. The disease may comprise an inflammatory disease. The inflammatory disease may be as defined above in relation to the fourth aspect.

In a further aspect, the present invention provides the use of a conjugate as defined in accordance with the first aspect in the manufacture of a medicament for the treatment of a disease in a mammalian subject. The disease may comprise cancer. The cancer may be as defined above in relation to the third aspect. The disease may comprise an inflammatory disease. The inflammatory disease may be as defined above in relation to the fourth aspect.

The invention includes the combination of the aspects and preferred features described except where such a combination is clearly impermissible or expressly avoided.

### Summary of the Figures

Embodiments and experiments illustrating the principles of the invention will now be discussed with reference to the accompanying figures in which:

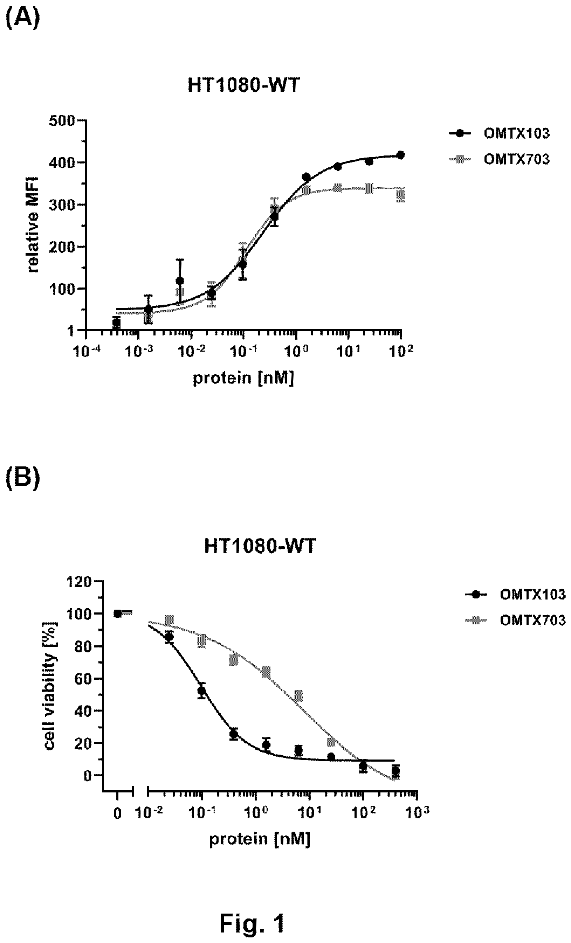

**Figure 1****. *In vitro* cell binding and cytotoxic assays for OMTX103.** (**A**) *In vitro* binding assay: binding affinity to HT1080-WT cells (measured as relative mean fluorescence intensity (MFI)) of anti-Endoglin ADCs, OMTX103 and OMTX703, measured against ADC concentration (nM). (**B**) *In vitro* cytotoxic assay: cell viability of HT1080-WT cells against concentration (nM) of anti-Endoglin ADCs, OMTX103 and OMTX703.

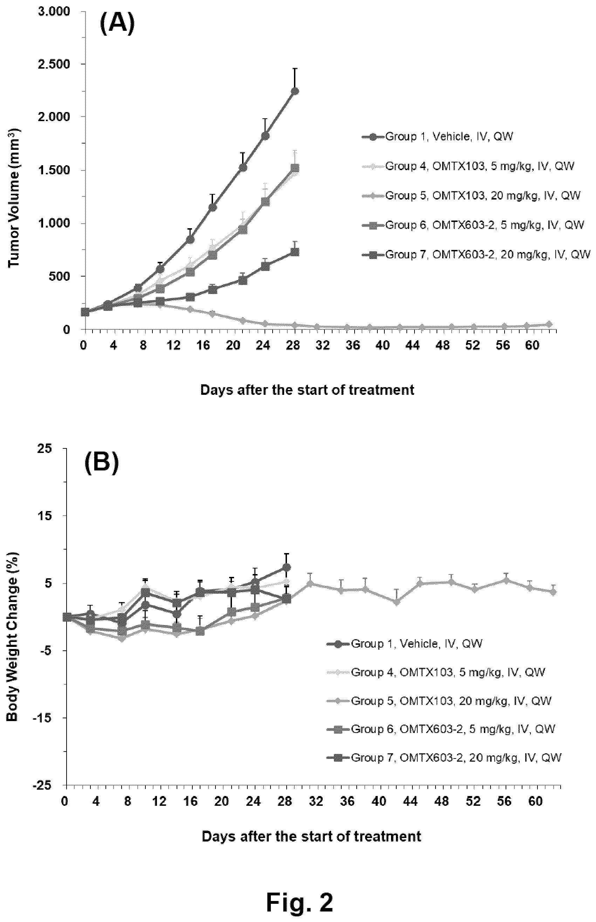

**Figure 2****. *In vivo* anti-tumoral efficacy of OMTX103 in a gastric cancer PDX model, ST-02-0318, with high expression of endoglin.** (**A**) Change in tumour volume (mm³) following administration at different weekly i.v. doses of OMTX103 (5 mg/kg and 20 mg/kg), compared to vehicle and OMTX603-2 (5 mg/kg and 20 mg/kg). (**B**) Patient change in body weight (%) of mice in (A) following the start of treatment.

### Detailed Description of the Invention

In describing the present invention, the following terms will be employed, and are intended to be defined as indicated below.

### Endoglin

As used "Endoglin" may be an Endoglin protein of any mammalian species. In some embodiments Endoglin is human Endoglin (also known as CD105, ENG or END), the amino acid sequence of which is disclosed at UniProt accession No. P17813 (Version 154, dated 13 November 2013) (SEQ ID NO: 25). In some embodiments, a molecule that binds Endoglin (e.g. an antibody molecule or a conjugate thereof) may bind to a region of the extracellular domain of Endoglin. The extracellular domain of human Endoglin comprises residues 26-561 of the full-length human Endoglin protein. In some embodiments Endoglin is murine Endoglin (also known as CD105, MJ7/18 antigen, ENG or END), the amino acid sequence of which is disclosed at UniProt accession No. Q63961 (Version 104, dated 13 November 2013) (SEQ ID NO: 26). The extracellular domain of murine Endoglin comprises residues 27-581 of the full-length murine Endoglin protein.

### Conjugate

As used herein "conjugate" includes the resultant structure formed by linking molecules and specifically includes antibody-drug conjugates (ADCs).

### Selectively binds

The terms selectively binds and selective binding refer to binding of an antibody, or binding fragment thereof, to a predetermined molecule (e.g. an antigen) in a specific manner. For example, the antibody, or binding fragment thereof, may bind to Endoglin, e.g. an extracellular portion thereof, with an affinity of at least about 1x10⁷M⁻¹, for example as measured at 25°C (e.g. measured by surface plasmon resonance(SPR), such as Biacore SPR). The antibody or binding fragment thereof may bind to the predetermined molecule (e.g. Endoglin) with an affinity that is at least two-fold greater (e.g. five-fold or ten-fold greater) than its affinity for binding to a molecule other than the predetermined molecule.

### Antibody molecule

As used herein with reference to all aspects of the invention, the term "antibody" or "antibody molecule" includes any immunoglobulin whether natural or partly or wholly synthetically produced. The term "antibody" or "antibody molecule" includes monoclonal antibodies (mAb) and polyclonal antibodies (including polyclonal antisera). Antibodies may be intact or fragments derived from full antibodies (see below). Antibodies may be human antibodies, humanised antibodies or antibodies of non-human origin. "Monoclonal antibodies" are homogeneous, highly specific antibody populations directed against a single antigenic site or "determinant" of the target molecule. "Polyclonal antibodies" include heterogeneous antibody populations that are directed against different antigenic determinants of the target molecule.

The term "antiserum" or "antisera" refers to blood serum containing antibodies obtained from immunized animals.

It has been shown that fragments of a whole antibody can perform the function of binding antigens. Thus reference to antibody herein, and with reference to the methods and compositions of the invention, covers a full antibody and also covers any polypeptide or protein comprising an antibody binding fragment. Examples of binding fragments are (i) the Fab fragment consisting of V_{L}, V_{H}, C_{L} and C_{H}1 domains; (ii) the Fd fragment consisting of the V_{H} and C_{H}1 domains; (iii) the Fv fragment consisting of the V_{L} and V_{H} domains of a single antibody; (iv) the dAb fragment which consists of a V_{H} domain; (v) isolated CDR regions; (vi) F(ab')₂ fragments, a bivalent fragment comprising two linked Fab fragments (vii) single chain Fv molecules (scFv), wherein a V_{H} domain and a V_{L} domain are linked by a peptide linker which allows the two domains to associate to form an antigen binding site; (viii) bispecific single chain Fv dimers (WO 93/11161) and (ix) "diabodies", multivalent or multispecific fragments constructed by gene fusion (WO94/13804; 58). Fv, scFv or diabody molecules may be stabilised by the incorporation of disulphide bridges linking the VH and VL domains. Minibodies comprising a scFv joined to a CH3 domain may also be made.

In relation to an antibody molecule, the term "selectively binds" may be used herein to refer to the situation in which one member of a specific binding pair will not show any significant binding to molecules other than its specific binding partner(s). The term is also applicable where e.g. an antigen-binding site is specific for a particular epitope that is carried by a number of antigens, in which case the specific binding member carrying the antigen-binding site will be able to bind to the various antigens carrying the epitope.

The antibody may be a fully human antibody.

The antibody may comprise a human monoclonal antibody.

For example, the antibody may comprise a human monoclonal antibody that:
(i) selectively binds human Endoglin and which comprises a heavy chain comprising the amino acid sequence of SEQ ID NO: 3 and a light chain comprising the amino acid sequence of SEQ ID NO: 4; or
(ii) selectively binds murine Endoglin and which comprises a heavy chain comprising the amino acid sequence of SEQ ID NO: 15 and a light chain comprising the amino acid sequence of SEQ ID NO: 16.

In some embodiments, the antibody comprises or consists of a human monoclonal antibody that selectively binds human Endoglin and which comprises a heavy chain comprising the amino acid sequence of SEQ ID NO: 3 and a light chain comprising the amino acid sequence of SEQ ID NO: 4.

### Pharmaceutical compositions

The conjugates of the present invention may be comprised in pharmaceutical compositions with a pharmaceutically acceptable excipient.

A pharmaceutically acceptable excipient may be a compound or a combination of compounds entering into a pharmaceutical composition which does not provoke secondary reactions and which allows, for example, facilitation of the administration of the conjugate, an increase in its lifespan and/or in its efficacy in the body or an increase in its solubility in solution. These pharmaceutically acceptable vehicles are well known and will be adapted by the person skilled in the art as a function of the mode of administration of the conjugate.

In some embodiments, conjugates of the present invention may be provided in a lyophilised form for reconstitution prior to administration. For example, lyophilised conjugates may be re-constituted in sterile water and mixed with saline prior to administration to an individual.

Conjugates of the present invention will usually be administered in the form of a pharmaceutical composition, which may comprise at least one component in addition to the conjugate. Thus pharmaceutical compositions may comprise, in addition to the conjugate, a pharmaceutically acceptable excipient, carrier, buffer, stabilizer or other materials well known to those skilled in the art. Such materials should be non-toxic and should not interfere with the efficacy of the conjugate. The precise nature of the carrier or other material will depend on the route of administration, which may be by bolus, infusion, injection or any other suitable route, as discussed below.

For intra-venous administration, e.g. by injection, the pharmaceutical composition comprising the conjugate may be in the form of a parenterally acceptable aqueous solution which is pyrogen-free and has suitable pH, isotonicity and stability. Those of relevant skill in the art are well able to prepare suitable solutions using, for example, isotonic vehicles, such as Sodium Chloride Injection, Ringer's Injection, Lactated Ringer's Injection. Preservatives, stabilizers, buffers, antioxidants and/or other additives may be employed as required including buffers such as phosphate, citrate and other organic acids; antioxidants, such as ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens, such as methyl or propyl paraben; catechol; resorcinol; cyclohexanol; 3'-pentanol; and m-cresol); low molecular weight polypeptides; proteins, such as serum albumin, gelatin or immunoglobulins; hydrophilic polymers, such as polyvinylpyrrolidone; amino acids, such as glycine, glutamine, asparagines, histidine, arginine, or lysine; monosaccharides, disaccharides and other carbohydrates including glucose, mannose or dextrins; chelating agents, such as EDTA; sugars, such as sucrose, mannitol, trehalose or sorbitol; salt-forming counter-ions, such as sodium; metal complexes (e.g. Zn-protein complexes); and/or non-ionic surfactants, such as TWEEN^{™}, PLURONICS^{™} or polyethylene glycol (PEG).

The phrase "pharmaceutically acceptable salt," as used herein, refers to pharmaceutically acceptable organic or inorganic salts of an ADC. Exemplary salts include, but are not limited to, sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate, and pamoate (i.e., 1,1'-methylene-bis-(2-hydroxy-3-naphthoate)) salts. A pharmaceutically acceptable salt may involve the inclusion of another molecule such as an acetate ion, a succinate ion or other counterions. The counterions may be any organic or inorganic ions that stabilize the charge on the parent compound. Furthermore, a pharmaceutically acceptable salt may have more than one charged atom in its structure. Instances where multiple charged atoms are part of the pharmaceutically acceptable salt can have multiple counter ions. Hence, a pharmaceutically acceptable salt can have one or more charged atoms and/or one or more counterion.

### Subject

The subject may be a human, a companion animal (e.g. a dog or cat), a laboratory animal (e.g. a mouse, rat, rabbit, pig or non-human primate), a domestic or farm animal (e.g. a pig, cow, horse or sheep). Preferably, the subject is a human. In some embodiments, the subject is a human diagnosed with or classified as being at risk of developing a cancer, e.g., an epithelial tumour, a solid tumour or a blood cancer. In certain embodiments the subject may be a laboratory animal, e.g., a mouse model of a cancer.

### Cancer

The anti-ENG conjugates described herein find particular use in a method of treatment of cancer in a mammalian subject. The cancer may comprise blood cancer. The cancer may comprise a solid tumour. The cancer may comprise gastric cancer, pancreatic cancer, breast cancer, melanoma, Ewing sarcoma, lung cancer, head & neck cancer, ovarian cancer, bladder cancer or colon cancer. The cancer may be an Endoglin positive cancer (including any of the above-described cancers) that expresses Endoglin on one or more cancer cells and/or on one or more cells forming part of the tumour microenvironment, in particular, one or more cells of the stroma. The cancer may be any stage. In particular, the cancer may be a metastatic cancer. In particular, the cancer may be gastric cancer that expresses Endoglin. The cancer may have been previously treated with one or more of chemotherapy, surgical resection, radiation therapy, immunotherapy or cell therapy. For example, the cancer may be a cancer in which a first line therapy has failed to prevent progression to a more advanced stage.

### Dosages

Multiple doses of the conjugate may be provided. One or more, or each, of the doses may be accompanied by simultaneous, sequential or separate administration of another anti-cancer agent.

In some embodiments the time interval between administration steps performed sequentially is one of at least 12 hours, 24 hours, 36 hours, 48 hours, 72 hours, 4 days, 5 days, 7 days, 10 days, 14 days, 18 days, 21 days, or 28 days, or 1, 2, 3, 4, 5, or 6 months. The conjugate may be administered at a dose equivalent to between 5 mg/kg IV and 50 mg/kg IV, e.g. between 10 mg/kg and 30 mg/kg or at approximately 20 mg/kg IV. Referring to Fig. 2(A), the tumour volume of the Group 5 mice (treated with a conjugate of the present invention at a dose of 20 mg/kg IV per week), was drastically reduced and maintained at a near zero volume for a substantial period of time (e.g. from around day 28 to beyond day 60). Without wishing to be bound by any particular theory, the inventors presently consider that a human dose equivalent to the murine dose of 20 mg/kg IV per week may show particular efficacy in the treatment of gastric cancer, among other forms of cancer.

In embodiments where administration steps comprise plural, separate introductions of material into a subject, time intervals provided herein may be between the final introduction of one administration step, and the first introduction of the subsequent administration step.

Preferably, the conjugate of the present invention, or a pharmaceutical composition comprising the conjugate according to the present invention is administered via injection. Such administration may be both via infusion (continuous) or bolus (discrete) administration. The method of administration via injection may be, for example, subcutaneous, intradermal, intramuscular, intravenous, intraarterial, intracardiac, intrathecal, intraspinal, intracapsular, subcapsular, intra-orbital, intraperitoneal, intratracheal, subcuticular, intraarticular, subarachnoid, and intra-sternal injection. Preferably, the administration is by intravenous infusion or intravenous injection (bolus administration). More preferably, the administration is by intravenous infusion.

Administration of the articles according to the present disclosure is preferably in a 'therapeutically-effective' or 'prophylactically-effective' amount, this being sufficient to show therapeutic/prophylactic benefit to the subject. The actual amount administered, and rate and time-course of administration, will depend on the nature and severity of the disease/condition and the particular article administered. Prescription of treatment, *e.g*. decisions on dosage *etc.*, is within the responsibility of general practitioners and other medical doctors, and typically takes account of the disease/disorder to be treated, the condition of the individual subject, the site of delivery, the method of administration and other factors known to practitioners. Examples of the techniques and protocols mentioned above can be found in Remington's 'The Science and Practice of Pharmacy' (ed. A. Adejare), 23rd Edition (2020), Academic Press.

### Combination therapy

The conjugate or composition of the present invention may be administered or be for administration simultaneously, sequentially or separately with one or more other anti-cancer agents. For example, the conjugate of the present invention, or a pharmaceutical composition comprising the conjugate according to the present invention, may be administered simultaneously, sequentially or separately with a chemotherapeutic agent or an immune checkpoint inhibitor. Examples of immune checkpoint inhibitors (ICls) (such as monoclonal antibodies) include PD-1 inhibitors, PD-L1 inhibitors, CTLA-4 inhibitors, LAG-3 inhibitors, and combinations thereof. In particular, an ICI may be selected from: ipilimumab, tislelizumab, tremelimumab, pembrolizumab, nivolumab, cemiplimab, atezolizumab, avelumab, durvalumab and relatlimab.

### Sequence identity

As used herein, 'sequence identity' refers to the percent of nucleotides/amino acid residues in a subject sequence that are identical to nucleotides/amino acid residues in a reference sequence, after aligning the sequences and, if necessary, introducing gaps, to achieve the maximum percent sequence identity between the sequences. Pairwise and multiple sequence alignment for the purposes of determining percent sequence identity between two or more amino acid or nucleic acid sequences can be achieved in various ways known to a person of skill in the art, for instance, using publicly available computer software such as ClustalOmega (Söding, J. 2005, Bioinformatics 21, 951-960), T-coffee (Notredame et al. 2000, J. Mol. Biol. (2000) 302, 205-217), Kalign (Lassmann and Sonnhammer 2005, BMC Bioinformatics, 6(298)) and MAFFT (Katoh and Standley 2013, Molecular Biology and Evolution, 30(4) 772-780) software. When using such software, the default parameters, e.g. for gap penalty and extension penalty, are preferably used.

### Isomers

Certain compounds of the invention may exist in one or more particular geometric, optical, enantiomeric, diasteriomeric, epimeric, atropic, stereoisomeric, tautomeric, conformational, or anomeric forms, including but not limited to, cis- and trans-forms; E- and Z-forms; c-, t-, and r- forms; endo- and exo-forms; R-, S-, and meso-forms; D- and L-forms; d- and I-forms; (+) and (-) forms; keto-, enol-, and enolate-forms; syn- and anti-forms; synclinal- and anticlinal-forms; α- and β-forms; axial and equatorial forms; boat-, chair-, twist-, envelope-, and halfchair-forms; and combinations thereof, hereinafter collectively referred to as "isomers" (or "isomeric forms").

The term "chiral" refers to a molecule with a non-superimposable mirror image. The term "achiral" refers to molecules which are superimposable on their mirror image partner.

The term "stereoisomers" refers to compounds which have the same molecular formula and sequence of bonded atoms, but differ in the three-dimensional orientation of their atoms in space.

"Diastereomer" refers to a stereoisomer with two or more centres of chirality and whose molecules are not mirror images of one another. Diastereomers have different physical properties, e.g. melting points, boiling points, spectral properties, and reactivities. Mixtures of diastereomers may separate under high resolution analytical procedures such as electrophoresis and chromatography.

"Enantiomers" refer to two stereoisomers of a compound which are non-superimposable mirror images of one another.

Stereochemical definitions and conventions used herein generally follow S. P. Parker, Ed., McGraw-Hill Dictionary of Chemical Terms (1984) McGraw-Hill Book Company, New York; and Eliel, E. and Wilen, S., "Stereochemistry of Organic Compounds", John Wiley & Sons, Inc., New York, 1994. The compounds of the invention may contain asymmetric or chiral centres, and therefore exist in different stereoisomeric forms. It is intended that all stereoisomeric forms of the compounds of the invention, including but not limited to, diastereomers, enantiomers and atropisomers, as well as mixtures thereof such as racemic mixtures, form part of the present invention, even if only one isomer is shown for a given compound or structure. Many organic compounds exist in optically active forms, i.e., they have the ability to rotate the plane of plane-polarized light. In describing an optically active compound, the prefixes D and L, or *R* and S, are used to denote the absolute configuration of the molecule about its chiral centre(s). The prefixes d and I or (+) and (-) are employed to designate the sign of rotation of plane-polarized light by the compound, with (-) or I meaning that the compound is levorotatory. A compound prefixed with (+) or d is dextrorotatory. For a given chemical structure, these stereoisomers are identical except that they are mirror images of one another. A specific stereoisomer may also be referred to as an enantiomer, and a mixture of such isomers is often called an enantiomeric mixture. A 50:50 mixture of enantiomers is referred to as a racemic mixture or a racemate, which may occur where there is no stereospecificity in a chemical reaction or process. The terms "racemic mixture" and "racemate" refer to an equimolar mixture of two enantiomeric species, devoid of optical activity.

"Enantiomerically enriched form" refers to a sample of a chiral substance whose enantiomeric ratio is greater than 50:50 but less than 100:0.

Note that, except as discussed below for tautomeric forms, specifically excluded from the term "isomers", as used herein, are structural (or constitutional) isomers (i.e. isomers which differ in the connections between atoms rather than merely by the position of atoms in space). For example, a reference to a methoxy group, -OCH₃, is not to be construed as a reference to its structural isomer, a hydroxymethyl group, -CH₂OH. Similarly, a reference to ortho-chlorophenyl is not to be construed as a reference to its structural isomer, meta-chlorophenyl. However, a reference to a class of structures may well include structurally isomeric forms falling within that class (e.g. C₁₋₇ alkyl includes n-propyl and iso-propyl; butyl includes n-, iso-, sec-, and tert-butyl; methoxyphenyl includes ortho-, meta-, and para-methoxyphenyl).

The above exclusion does not pertain to tautomeric forms, for example, keto-, enol-, and enolate-forms, as in, for example, the following tautomeric pairs: keto/enol (illustrated below), imine/enamine, amide/imino alcohol, amidine/enediamine, nitroso/oxime, thioketone/enethiol, and N-nitroso/hyroxyazo.

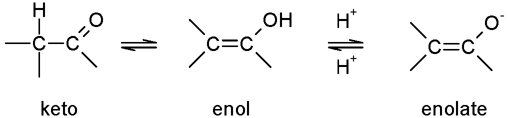

The term "tautomer" or "tautomeric form" refers to structural isomers of different energies which are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons. Accordingly, the compounds according to the present invention include all tautomeric forms even if only one is shown.

The compounds according to the present invention include all isotopic forms even if only one is shown, i.e., compounds with one or more isotopic substitutions. For example, H may be in any isotopic form, including ¹H, ²H (D), and ³H (T); C may be in any isotopic form, including ¹²C, ¹³C, and ¹⁴C; O may be in any isotopic form, including ¹⁶O and ¹⁸O; and the like.

Examples of isotopes that can be incorporated into compounds of the invention include isotopes of hydrogen, carbon, nitrogen, oxygen, phosphorous, fluorine, chlorine and iodine, such as, but not limited to ²H (deuterium, D), ³H (tritium), ¹¹C, ¹³C, ¹⁴C, ¹⁵N, ¹⁸F, ³¹P, ³²P, ³⁵S, ³⁶Cl, and ¹²⁵I. Various isotopically labelled compounds of the present invention, for example those into which radioactive isotopes such as ³H, ¹³C, and ¹⁴C are incorporated. Such isotopically labelled compounds may be useful in metabolic studies, reaction kinetic studies, detection or imaging techniques, such as positron emission tomography (PET) or single-photon emission computed tomography (SPECT) including drug or substrate tissue distribution assays, or in radioactive treatment of patients. Deuterium labelled or substituted therapeutic compounds of the invention may have improved DMPK (drug metabolism and pharmacokinetics) properties, relating to distribution, metabolism, and excretion (ADME). Substitution with heavier isotopes such as deuterium may afford certain therapeutic advantages resulting from greater metabolic stability, for example increased *in vivo* half-life or reduced dosage requirements. An ¹⁸F labelled compound may be useful for PET or SPECT studies. Isotopically labelled compounds of this invention and prodrugs thereof can generally be prepared by carrying out the procedures disclosed in the schemes or in the examples and preparations described below by substituting a readily available isotopically labelled reagent for a non-isotopically labelled reagent. Further, substitution with heavier isotopes, particularly deuterium (i.e., ²H or D) may afford certain therapeutic advantages resulting from greater metabolic stability, for example increased in vivo half-life or reduced dosage requirements or an improvement in therapeutic index. It is understood that deuterium in this context is regarded as a substituent. The concentration of such a heavier isotope, specifically deuterium, may be defined by an isotopic enrichment factor. In the compounds of this invention any atom not specifically designated as a particular isotope is meant to represent any stable isotope of that atom.

Unless otherwise specified, a reference to a particular compound includes all such isomeric forms, including (wholly or partially) racemic and other mixtures thereof. Methods for the preparation (e.g. asymmetric synthesis) and separation (e.g. fractional crystallisation and chromatographic means) of such isomeric forms are either known in the art or are readily obtained by adapting the methods taught herein, or known methods, in a known manner.

### Conjugates According to Formula (I)

Provided herein is an antibody-drug conjugate having a structure of Formula (I), or a pharmaceutically acceptable salt or solvate thereof: 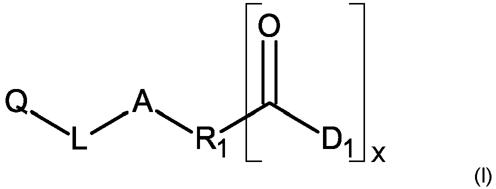 wherein:
**Q** is an antibody that selectively binds Endoglin;
L is a linker;
A is a bond or 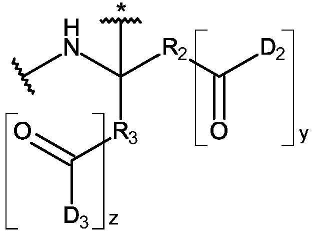 where the asterisk (*) marks the point of attachment to R₁;
each of D₁, D₂ and D₃ are independently MMAE;
each of x, y and z is independently 0 or 1, with a provision that at least one of x, y, and z is 1;
each of R₁, R₂, and R₃ is independently selected from: a bond, hydrogen, 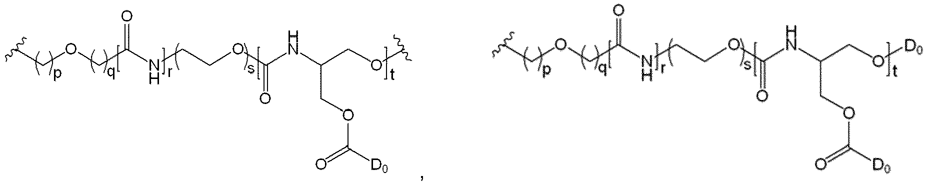 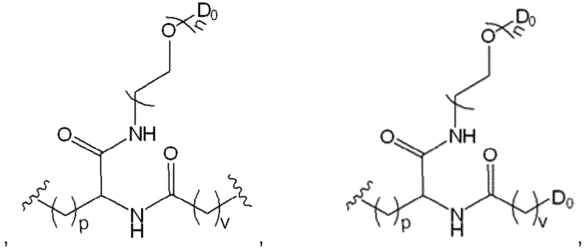 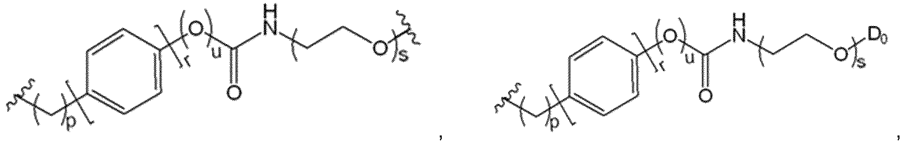 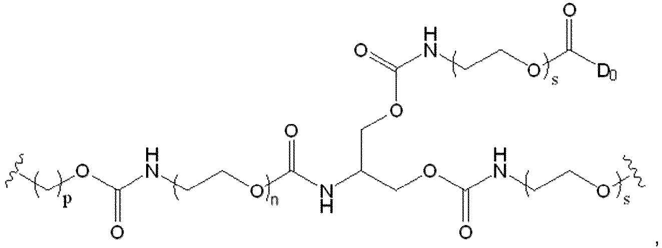 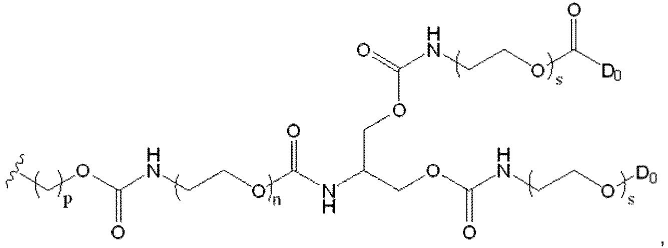 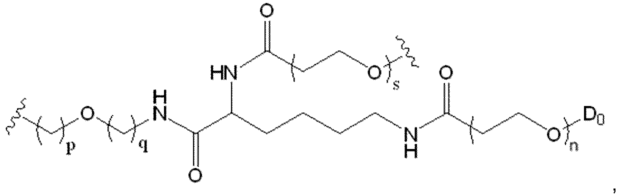 and 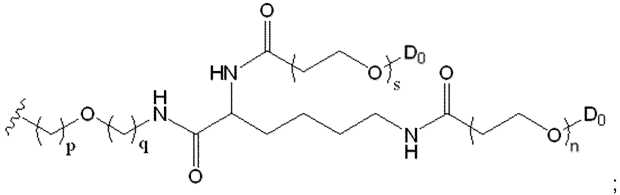
wherein:
   p is 1, 2, or 3;
   q is 0, 1, or 2;
   r is 0 or 1;
   s is an integer from 0 to 37;
   t is 0 or 1;
   u is 0 or 1;
   v is 1, 2, or 3; and
   n is an integer from 1 to 37; or
   x and y are 0, and R₁ and R₂ together form =O and R₃ is 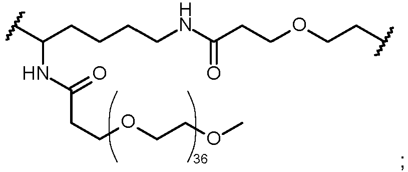
   D₀ is methyl, propargyl, or MMAE;
   with a provision that at least one of R₁, R₂, and R₃ is not hydrogen;
   if q, r, s, and t are 0, then x + y + z > 1;
   if x is 0, then R₁ is selected from: hydrogen, 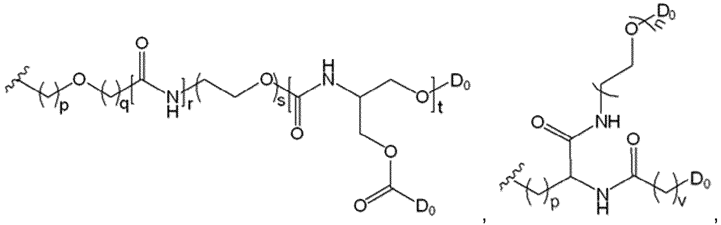 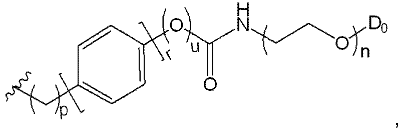 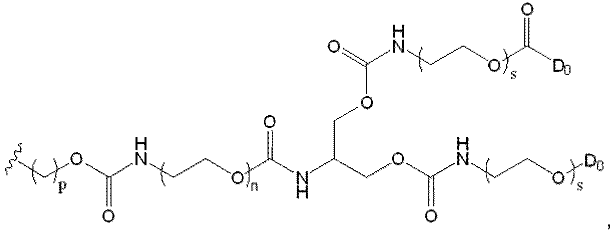 and 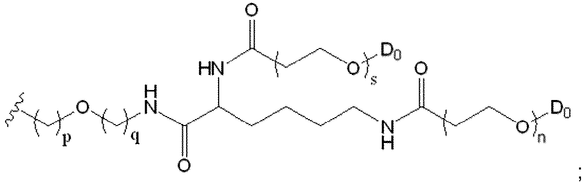
   if y is 0, then R₂ is selected from: hydrogen, 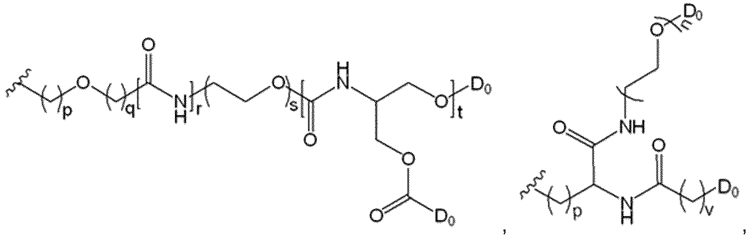 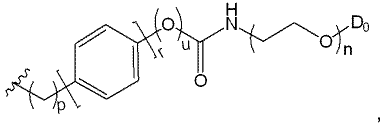 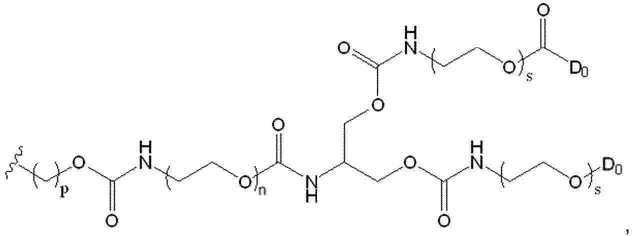 and 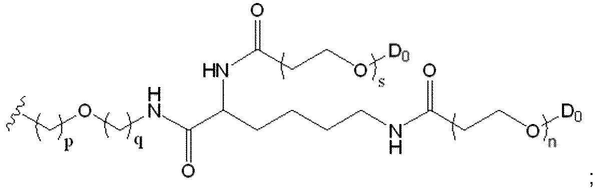
   if z is 0, then R₃ is selected from: hydrogen, 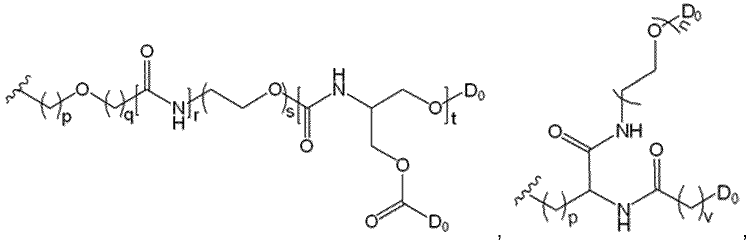 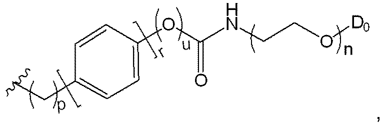 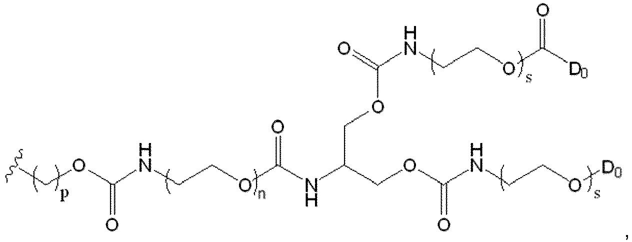 and 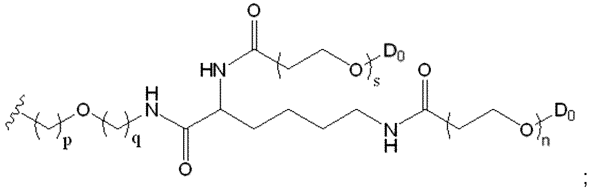
   if x + y + z = 1, and each of R₁, R₂, and R₃ independently is 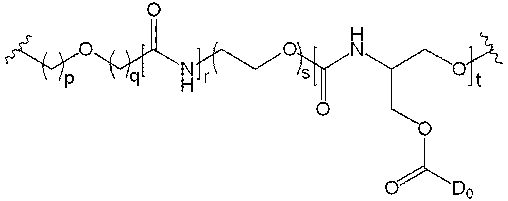 or 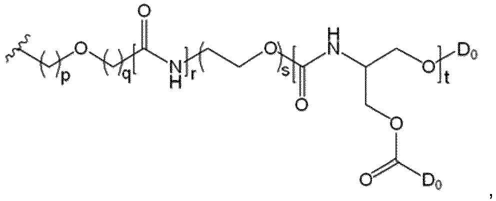 then p is not 1; and
   if each of R₁, R₂, and R₃ independently is 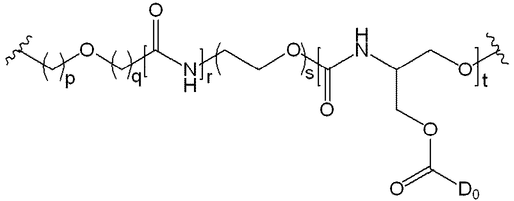 or 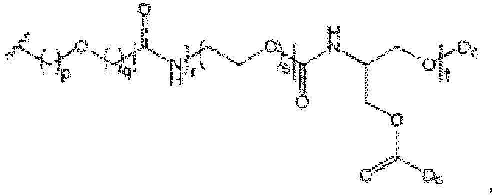 and r, s, and t are each 0,
      then x + y + z > 1.

Also provided herein is an antibody-drug conjugate having a structure of Formula (I), or a pharmaceutically acceptable salt or solvate thereof: 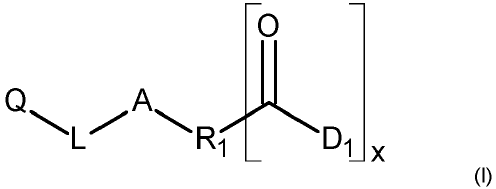 wherein:
**Q** is an antibody that selectively binds Endoglin;
L is a linker;
A is a bond or 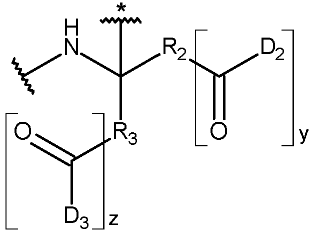 where the asterisk (*) marks the point of attachment to R₁;
each of D₁, D₂ and D₃ (where present) are independently MMAE;
each of x, y and z is independently 0 or 1, with a provision that at least one of x, y, and z is 1;
each of R₁, R₂, and R₃ is independently selected from: a bond, hydrogen, 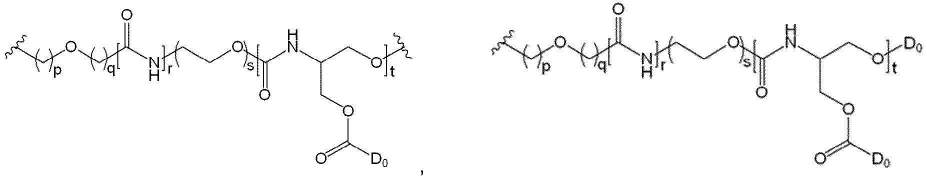 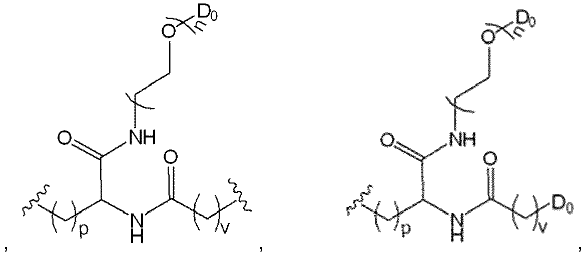 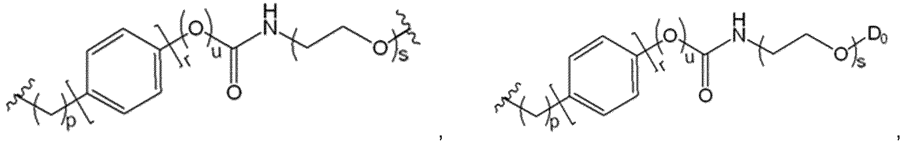 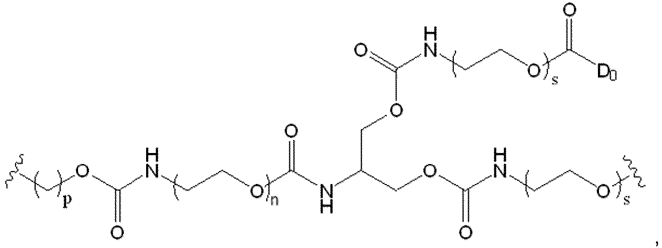 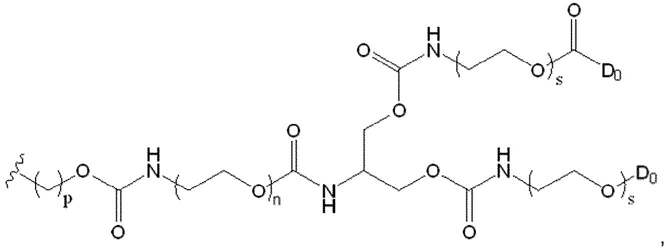 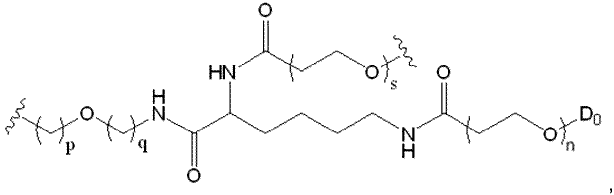 and 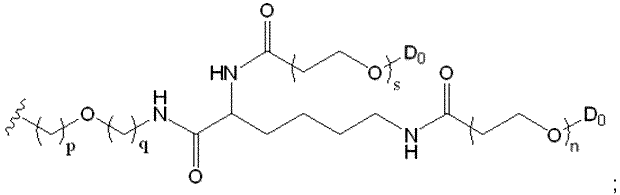 wherein:
   p is 1, 2, or 3;
   q is 0, 1, or 2;
   r is 0 or 1;
   s is an integer from 0 to 37;
   t is 0 or 1;
   u is 0 or 1;
   v is 1, 2, or 3; and
   n is an integer from 1 to 37; or
   x and y are 0, and R₁ and R₂ together form =O and R₃ is 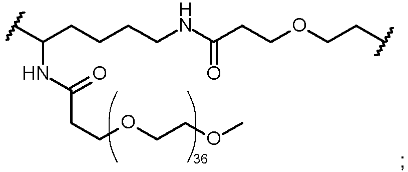
   D₀ is methyl, propargyl, or MMAE; and
   optionally, if q, r, s, and t are 0, then x + y + z > 1.

In some preferred embodiments, the conjugate has a structure according to Formula (II), or a pharmaceutically acceptable salt of solvate thereof: 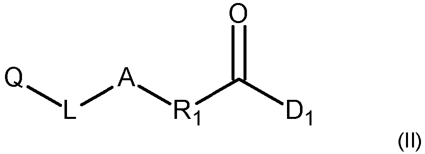 wherein:
**Q** is an antibody that selectively binds Endoglin;
L is a linker;
A is a bond or -NH-CH₂-;
D₁ is MMAE;
R₁ is selected from: a bond, 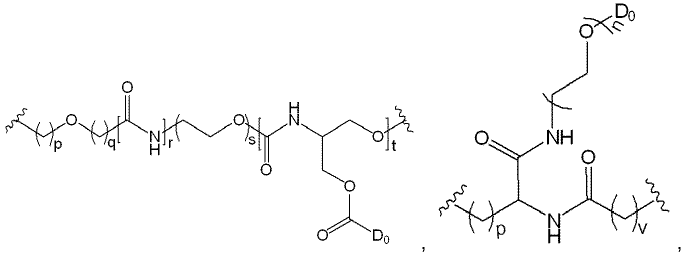 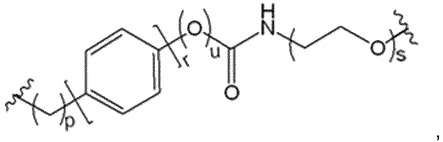 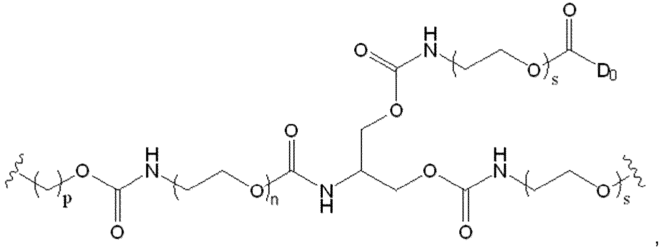 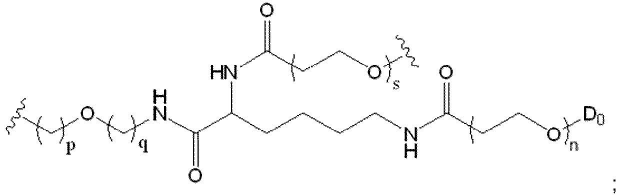
wherein:
   p is 1, 2, or 3;
   q is 0, 1, or 2;
   r is 0 or 1;
   s is an integer from 0 to 37;
   t is 0 or 1;
   u is 0 or 1;
   v is 1, 2, or 3; and
   n is an integer from 1 to 37; and
   if R₁ is 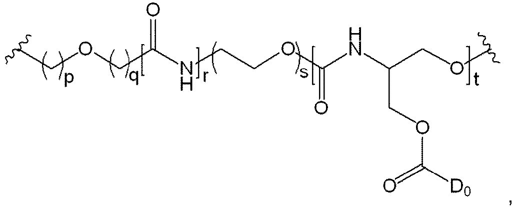 then p is not 1.

In some embodiments of Formula (II), if present, q + r + s + t > 0. In some embodiments, at least one of q, r, s, and t, if present, is not zero.

### Payload

In the conjugate of the present invention, the payload generally is monomethyl auristatin E (MMAE), or a pharmaceutically acceptable salt or solvate thereof. MMAE has IUPAC name (2S)-N-[(2S)-1-[[(3R,4S,5S)-1-[(2S)-2-[(1R,2R)-3-[[(1S,2R)-1-hydroxy-1-phenylpropan-2-yl]amino]-1-methoxy-2-methyl-3-oxopropyl]pyrrolidin-1-yl]-3-methoxy-5-methyl-1-oxoheptan-4-yl]-methylamino]-3-methyl-1-oxobutan-2-yl]-3-methyl-2-(methylamino)butanamide. It has CAS Registry Number 474645-27-7.

The linker-payload moiety comprises at least one MMAE payload, which may be represented by one or more of D₁, D₂, and D₃ in Formula (I). In some embodiments, the linker-payload moiety comprises more than one MMAE payload. In some preferred embodiments, the linker-payload moiety comprises only one MMAE payload.

In Formula (I), each of D₁, D₂ and D₃, when present, are MMAE. Additionally, in Formula (I), D₀, when present, may be MMAE.

MMAE has the structure:

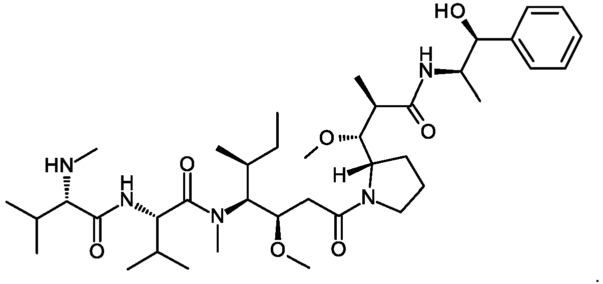

MMAE forms a bond to the rest of the conjugate (e.g., through one or more of carbonyl, bridging group (A), spacer (e.g. R₁), and linker (L)) via its terminal methyl amine. That is, in the below structure, the asterisk (*) marks the point of attachment to the rest of conjugate (e.g. to R₁ (via C(O))):

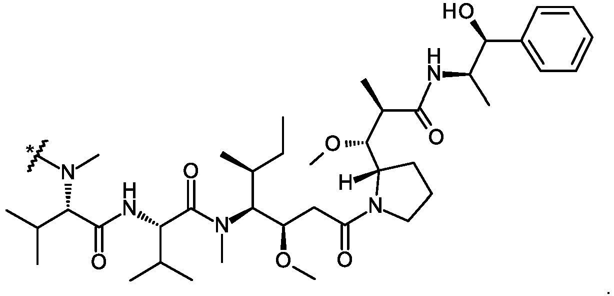

### Linker, L

The term "linker" as used herein refers to a component linking the antibody to the rest of the conjugate and the drug. That is, the linker according to the present invention refers to a component of the conjugate of the present invention that connects the anti-Endoglin antibody and the MMAE payload, optionally via one or more of a bridging group and/or spacer group. That is, the conjugates of the present invention may also comprise a bridging group, A, and one, two, or three spacer groups, R₁, R₂, R₃, which additionally form part of the connection between the antibody and the payload. In some embodiments, the bridging group and/or the spacer group(s) are a bond (i.e. they can be considered absent).

In some embodiments, the linker according to the present invention is a cleavable linker. Cleavable linkers are cleaved to release the drug payload (*i.e*. the MMAE) to the target location (*i.e*. the target cell, tissue *etc.).*

In some embodiments, the linker according to the present invention is an enzymatically cleavable linker. In some embodiments, the linker is a peptide-based linker. In some embodiments, the linker is a protease-cleavable linker, optionally a lysosomal protease-cleavable linker. In some embodiments, the linker is cleaved by an intracellular protease. In some embodiments, the linker is cleaved by a cathepsin. In some embodiments, the linker according to the present invention comprises a valine-citrulline unit. Valine-citrulline (Val-Cit) is a protease sensitive dipeptide that is cleaved intracellularly by cathepsins (*i.e*. cathepsin B).

In some embodiments, the linker according to the present invention comprises a self-immolative group. That is, in some embodiments, the linker comprises a group that undergoes self-immolative elimination to facilitate release of the drug payload (*i.e*. the MMAE) to the target location (*i.e*. the target cell, tissue *etc.).* In some embodiments, the linker comprises a para-aminobenzylcarbamate unit.

In some embodiments, the linker is coupled to the antibody via cysteine-based conjugation. In some embodiments, the linker comprises a thiol-reactive group. In some embodiments, the linker is coupled to the antibody via reaction between cysteine residues in the antibody and a thiol-reactive functional group in the linker. In some embodiments the linker comprises a maleimide group. In some embodiments, the linker comprises a maleimidocaproyl group. The maleimidocaproyl group according to the present invention may be reacted with a thiol group of a cysteine residue of the antibody according to the present invention to form a sulphur-carbon bond, thereby effecting linkage of the linker to the antibody.

In Formula (I), the linker, L, connects the anti-Endoglin antibody, Q, to bridging group, A.

The linker is conjugated to an amino acid residue on the antibody.

In some embodiments, L is selected from the group consisting of: 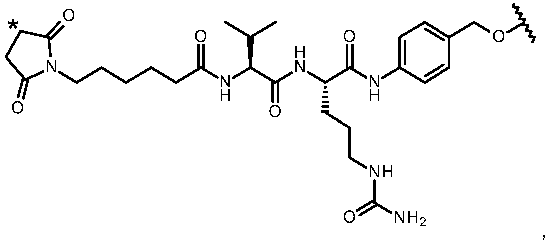 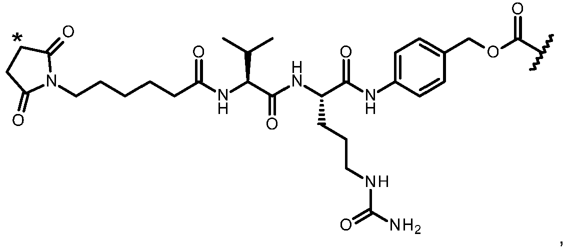 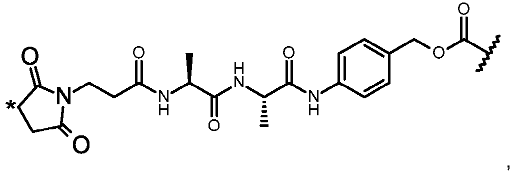 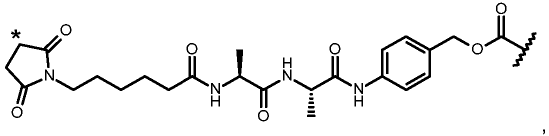 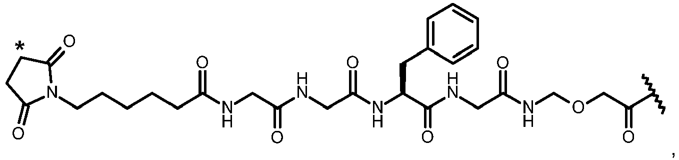 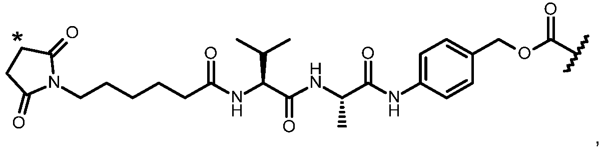 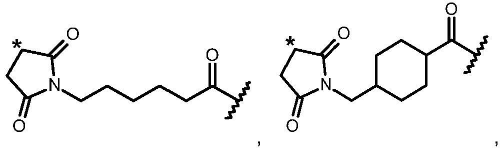 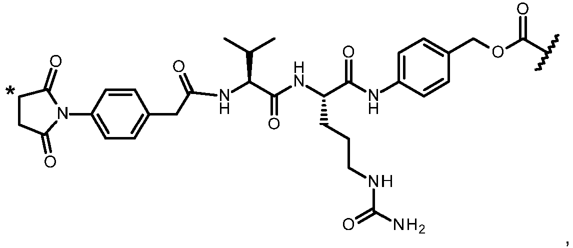 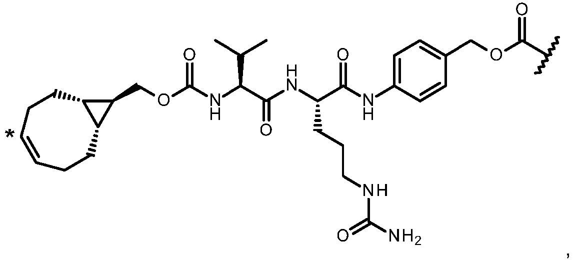 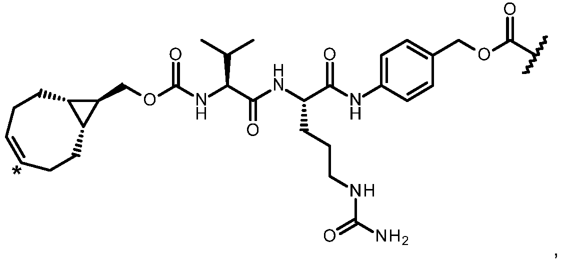 and 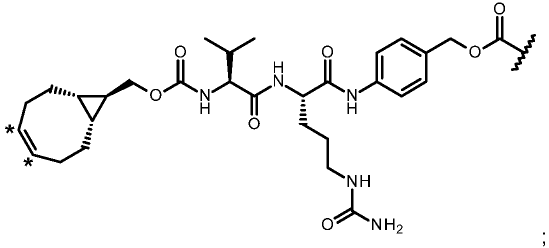 where the asterisk (*) marks the point of attachment to the antibody Q.

Where the point of attachment is depicted as being at the 3-position on the maleimidyl group, the point of attachment may equally be at the 4-position. That is, the below groups are considered equivalent: 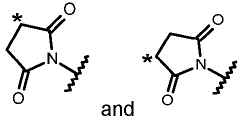 where the asterisk (*) marks the point of attachment to the antibody Q.

In some preferred embodiments, L is selected from the group consisting of: 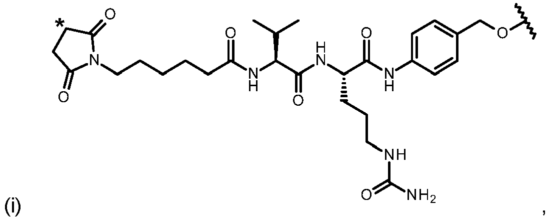 or 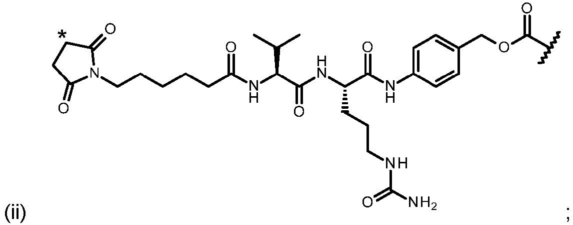 where the asterisk (*) marks the point of attachment to Q.

In some preferred embodiments, L has structure (i): 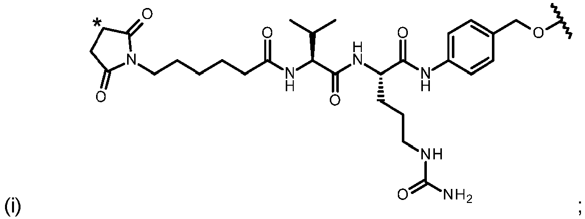 wherein the asterisk (*) marks the point of attachment to the antibody Q.

In some preferred embodiments, L has structure (ii): 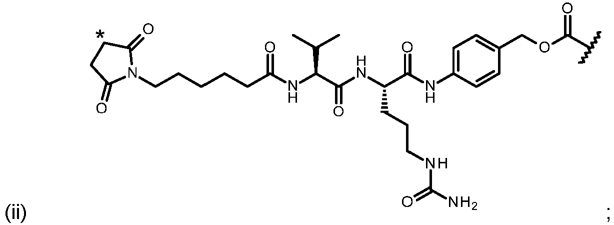 wherein the asterisk (*) marks the point of attachment to the antibody Q.

In some embodiments, L comprises the structure:

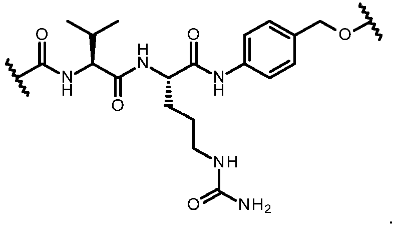

In some embodiments, L comprises the structure: 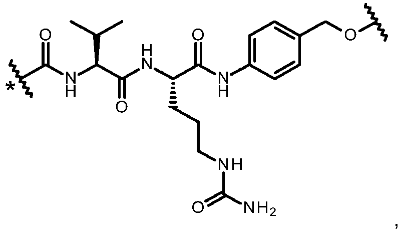 wherein a conjugating group is connected (optionally via a joining group) at the asterisk (*). The conjugating group is a group which conjugates the linker-payload to the antibody; optionally the conjugating group is a maleimide group; further optionally the joining group is an alkylene group (e.g., - (CH₂)₅-).

### Linker-Payload Moieties

The conjugate of the present invention comprises a linker-payload moiety conjugated to an antibody. The linker-payload moiety comprises a linker, L; a bridging group, A; one, two, or three spacer groups, R₁, R₂, and R₃; and a payload represented by one or more of D₁, D₂, and D₃. The bridging group may be a bond (i.e., effectively being absent). One or more of the spacer groups may be a bond or a hydrogen (i.e., effectively being absent).

There may be from 1 to 6 payloads on a linker-payload moiety. Preferably, there are from 1 to 3 payloads. More preferably, there are 1 or 2 payloads, and most preferably, there is 1 payload on a linker-payload moiety.

The linker-payload moiety has structure L-A-R₁-[C(O)-D₁]ₓ, where L, A, R₁, D₁ and x are as defined in Formula (I).

In some preferred embodiments, the linker-payload moiety has the structure L-C(O)-D₁ or L-NH-CH₂-C(O)-D₁. In some embodiments, the linker-payload moiety has the structure L-C(O)-D₁. In some embodiments, the linker-payload moiety has the structure L-NH-CH₂-C(O)-D₁.

In some preferred embodiments, the linker-payload moiety has the structure: 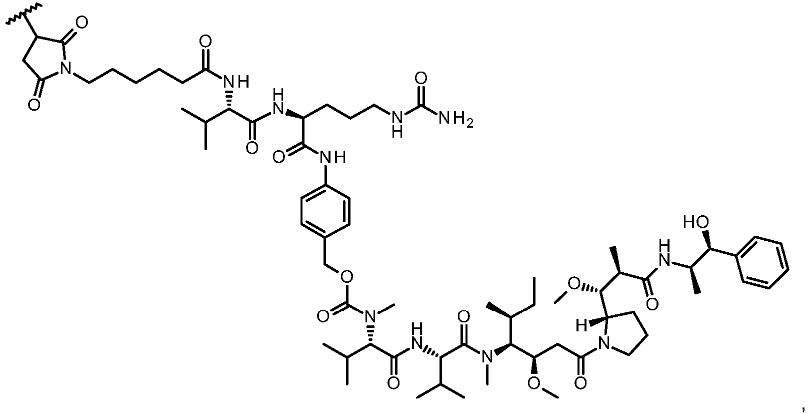 or

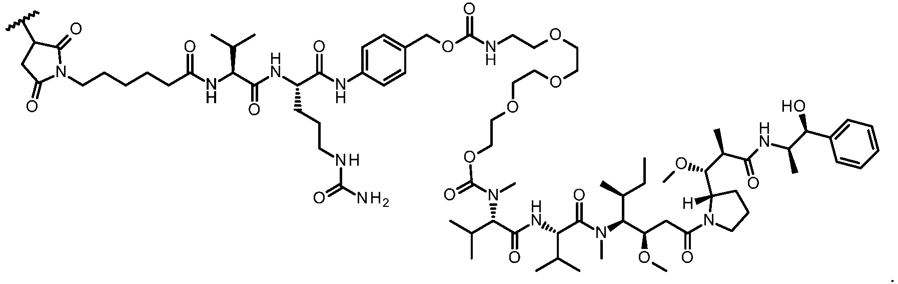

In some embodiments, the linker-payload moiety has the structure:

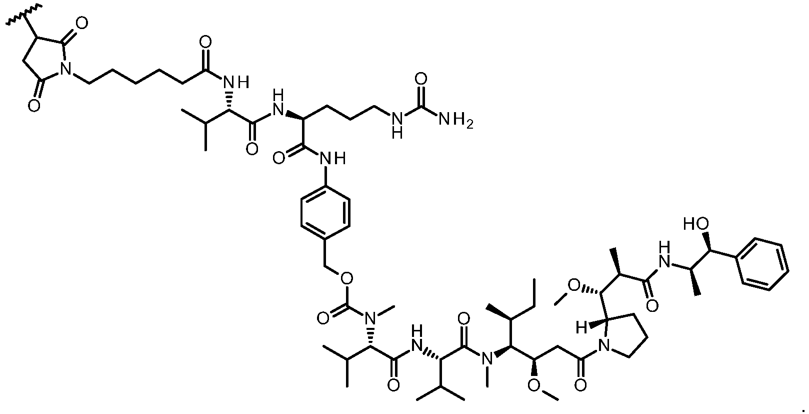

In some embodiments, the linker-payload moiety has the structure:

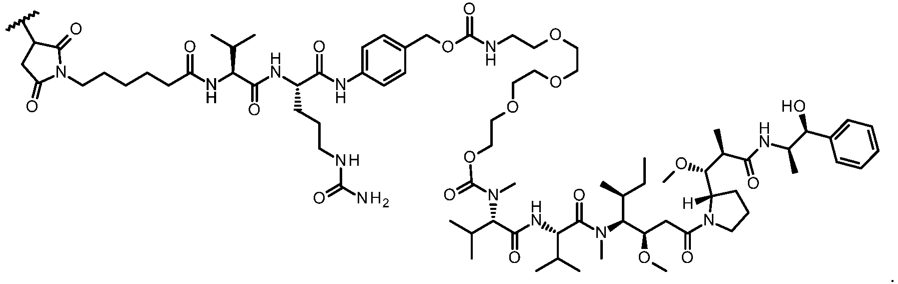

### x, y, and z

x, y, and z are indices in Formula (I) to indicate the presence or absence of a -C(O)-Dᵢ group (where i = 1, 2, 3). When x/y/z is 1, the corresponding -C(O)-Dᵢ group is present. When x/y/z is 0, the corresponding - C(O)-Dᵢ group is absent.

x, y, and z are each independently 0 or 1, with the proviso that at least one of x, y, and z is 1.

In some embodiments, x + y + z = 1 or 2. In some embodiments, x + y + z = 1.

In some embodiments, x is 1.

In some embodiments, x is 1, y is 0, and z is 0.

### Bridging Group, A

The bridging group is represented by A in Formula (I).

A is either a bond or has the structure: 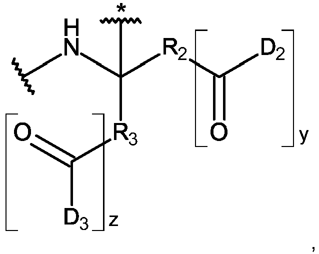 where the asterisk (*) marks the point of attachment to R₁, and where R₂, R₃, D₂, D₃, z and y are as defined in Formula (I).

In some embodiments, A is a bond. That is to say, when A is a bond, L bonds directly to R₁.

In cases where A is a bond, x in Formula (I) is 1.

In cases where x in Formula (I) is 0, A cannot be a bond.

In some embodiments, (such as those where x in Formula (I) is 0), A has the structure: 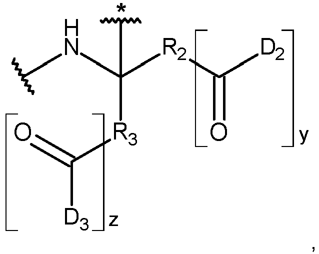 where the asterisk (*) marks the point of attachment to R₁, and where R₂, R₃, D₂, D₃, z and y are as defined in Formula (I).

In some embodiments, A is -NH-C(R₂)(R₃)- (i.e., when z and y both equal 0).

In some embodiments, A is -NH-CH₂- (i.e., when z and y both equal 0, and when R₂ and R₃ are both H).

### Spacer Groups, R₁, R₂, R₃

Spacer groups, R₁, R₂, and R₃ may be present and attached to the bridging group, A. The payload may be attached to the bridging group, A (and hence to the linker, L) via a spacer group, R₁, R₂, or R₃. For example, when MMAE is present as D₁, it is connected to the bridging group, A, via -R₁-C(O)-.

Each of R₁, R₂, and R₃ is independently selected from: a bond, hydrogen, 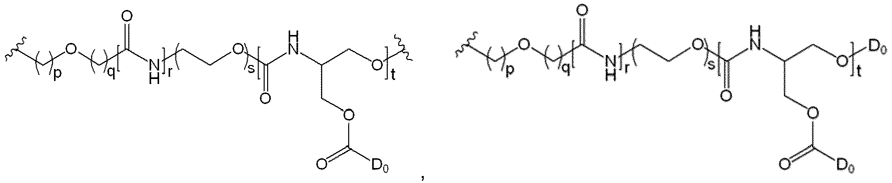 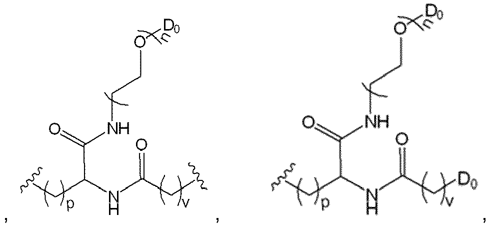 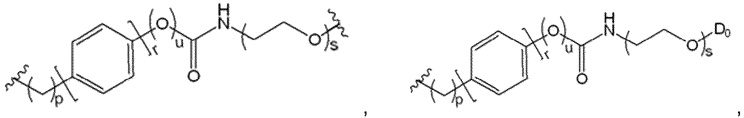 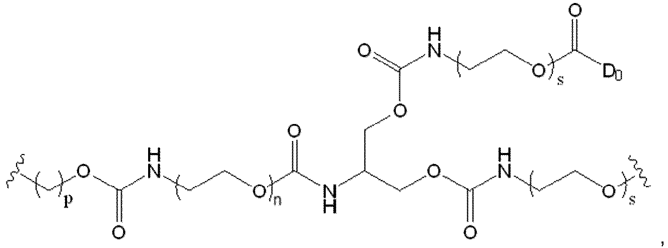 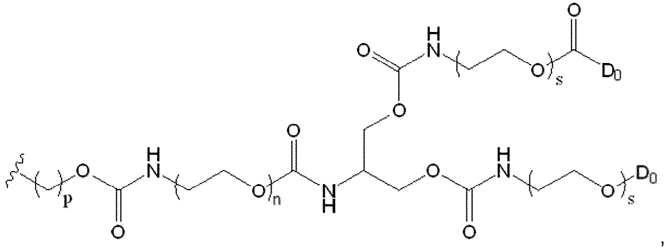 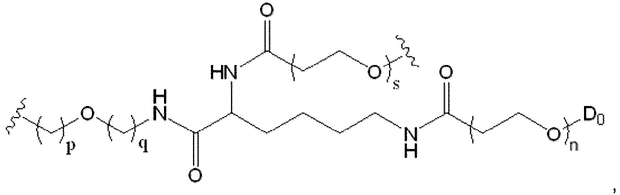 and 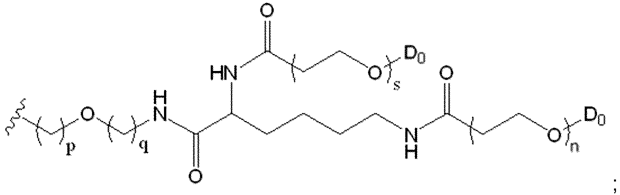 where D₀ is methyl, propargyl, or MMAE; p is 1, 2, or 3; q is 0, 1, or 2; r is 0 or 1; s is an integer from 0 to 37; t is 0 or 1; u is 0 or 1; v is 1, 2, or 3; and n is an integer from 1 to 37.

For the avoidance of doubt, the 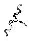 on the far left of each above group represents the connection to A.

When x, y, or z is 0, the respective spacer group (R₁, R₂, R₃) is a monovalent group selected from the above list. When x, y, or z is 1, the respective spacer group (R₁, R₂, R₃) is a bond or a bivalent group selected from the above list.

If x is 0, then R₁ is selected from: hydrogen, 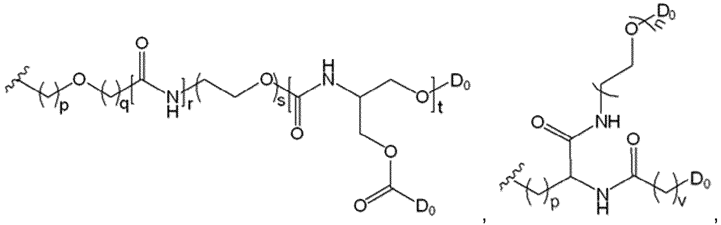 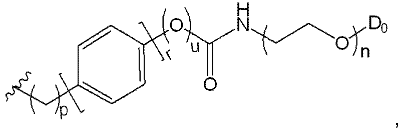 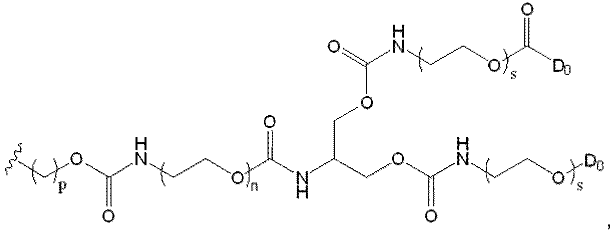 and

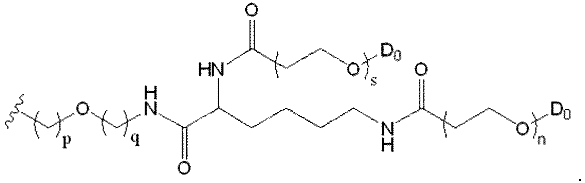

If y is 0, then R₂ is selected from: hydrogen, 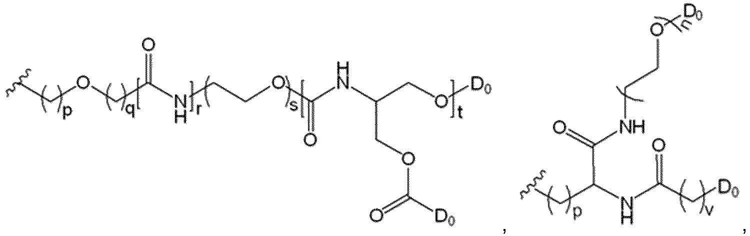 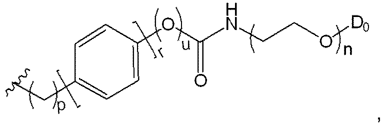 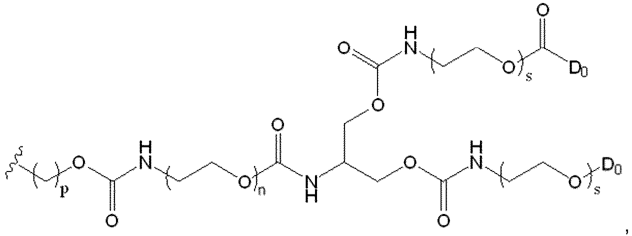 and

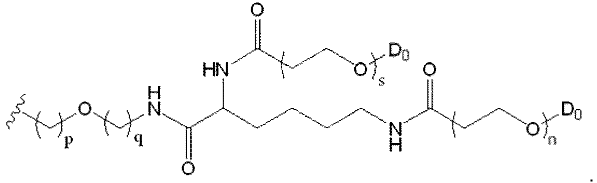

If z is 0, then R₃ is selected from: hydrogen, 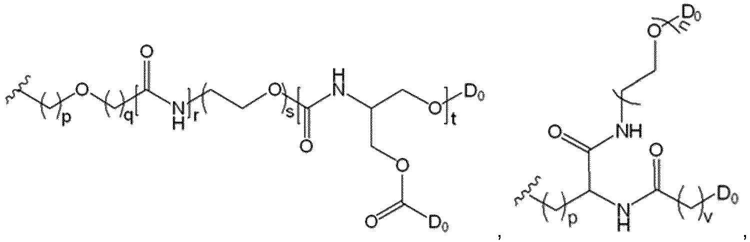 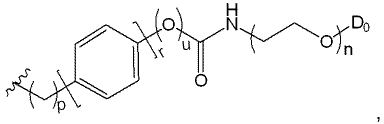 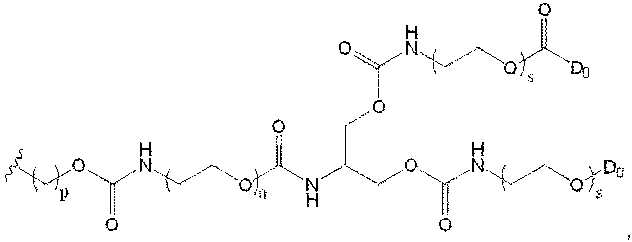 and

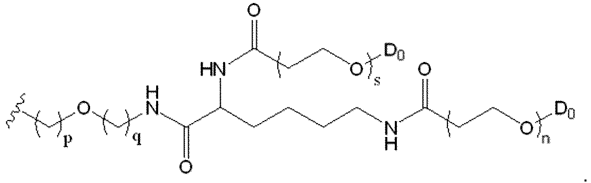

Optionally, if x + y + z = 1, and each of R₁, R₂, and R₃ independently is 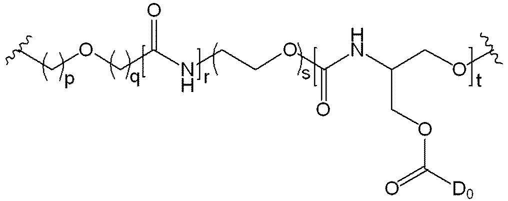 or 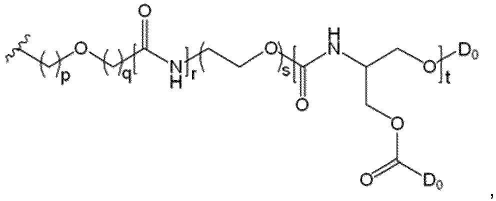 then p is not 1.

Optionally, if each of R₁, R₂, and R₃ independently is 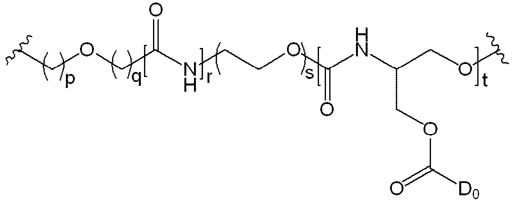 or 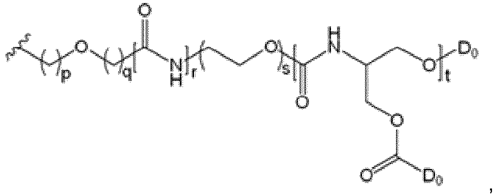 and if r, s, and t are each 0, then x + y + z > 1.

Optionally, if x+y+z=1 and each of R₁, R₂, and R₃ independently is 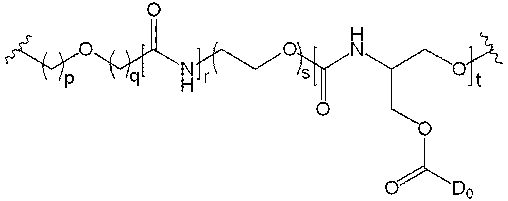 or 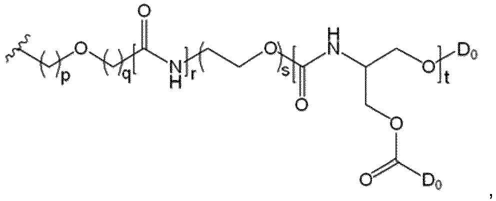 then p is not 1.

Optionally, if only one of x, y and z is 1, and the respective R₁, R₂ or R₃ is 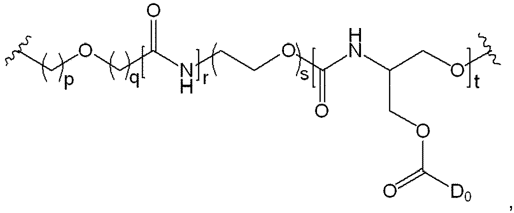 and if the remaining two of x, y, and z are 0 and the respective R₁, R₂ or R₃ is 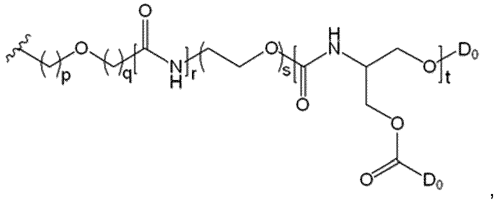 then p is not 1.

Optionally, if r, s, and t are 0 and each of R₁, R₂, and R₃ independently is 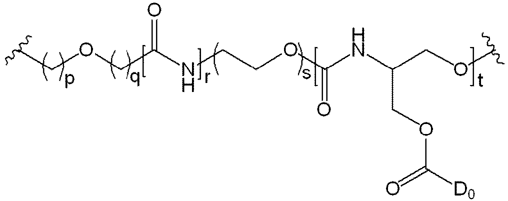 or 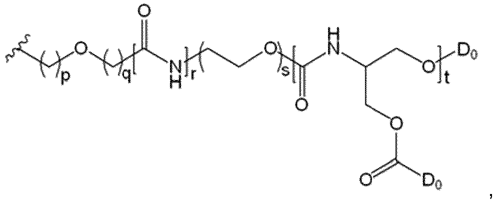 then x+y+z>1.

In some embodiments, one or more of R₁, R₂ and R₃ is a bond or

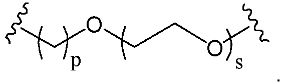

In some embodiments, R₁, R₂ and R₃ is each independently selected from: a bond, hydrogen, or

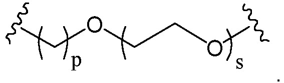

In some embodiments, one or more of R₁, R₂ and R₃ is a bond (and the respective x, y, z is 1). In some embodiments, R₁ is a bond (and x is 1). In some embodiments, R₁ is a bond (and x is 1), and y and z are both 0. In some embodiments, R₁ is a bond, and R₂ and R₃ are both H. In some embodiments, R₁ is a bond, R₂ is H, and R₃ is H.

In some preferred embodiments, R₁ is a bond and A is a bond. That is, L is bonded directly to -C(O)-D₁. That is, the conjugate has the structure Q-L-C(O)-D₁, with Q, L, and D₁ as defined in Formula (I).

In some preferred embodiments, R₁ is a bond, A is a bond, and L has structure: 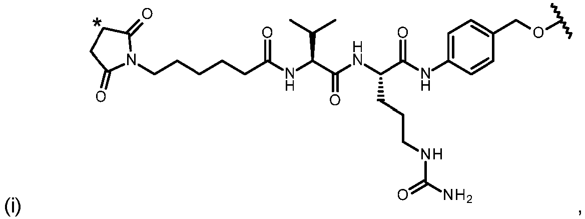 where the asterisk (*) marks the point of attachment to Q.

In some embodiments, one or more of R₁, R₂ and R₃ is

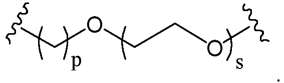

In some embodiments, R₁ is

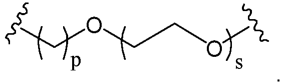

In some preferred embodiments, R₁ is 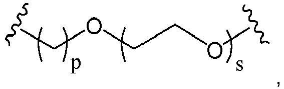 and y and z are both 0.

In some preferred embodiments, R₁ is 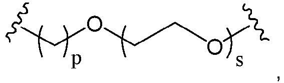 and R₂ and R₃ are both H.

In some embodiments, R₁ is 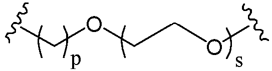 and A is -NH-CH₂-.

In some preferred embodiments, R₁ is 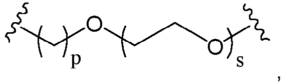 A is -NH-CH₂-, and L has structure: 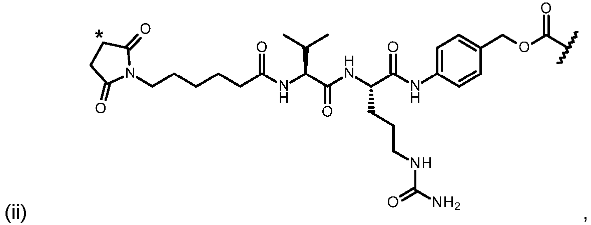 where the asterisk (*) marks the point of attachment to Q.

In some embodiments, R₁ is 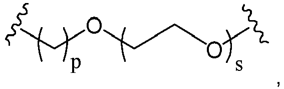 where p is 1 and s is 3.

In some preferred embodiments, R₁ is

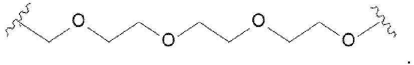

In some preferred embodiments, R₁ is 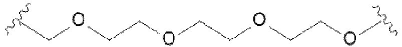 and A is -NH-CH₂-.

In some preferred embodiments, R₁ is 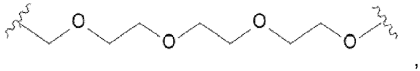 A is -NH-CH₂-, and L has structure: 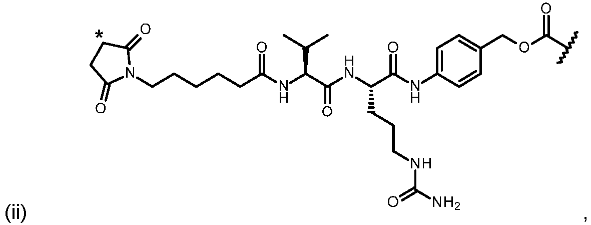 where the asterisk (*) marks the point of attachment to Q.

### Preferred Structures

The conjugate of the present invention may have one of the following most preferred structures.

In some embodiments, the ADC has the structure: 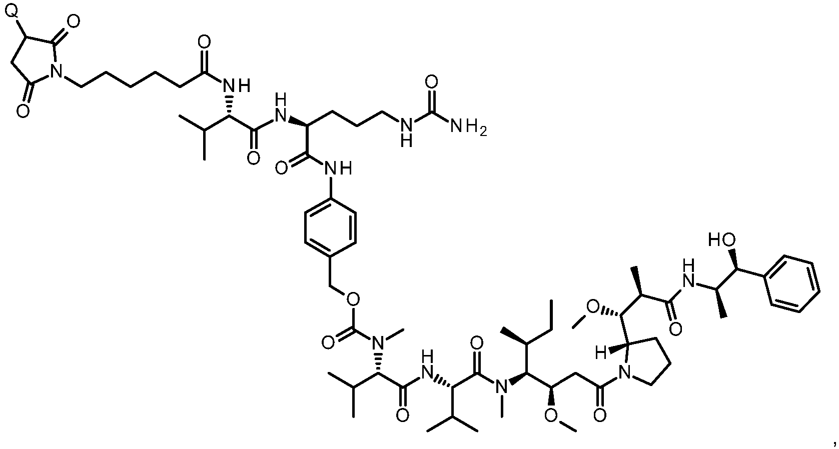 or

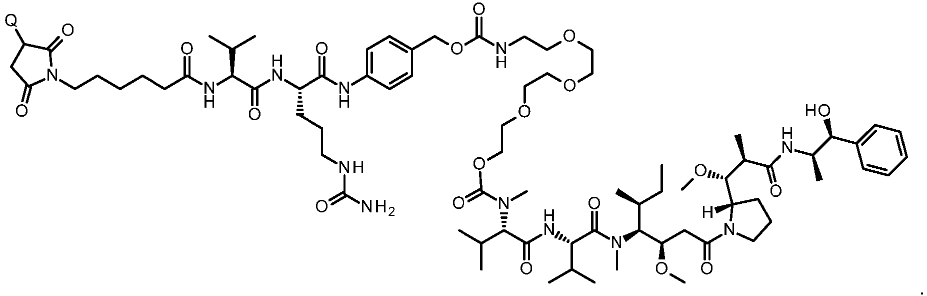

In some embodiments, the ADC is OMTX103, having the structure: 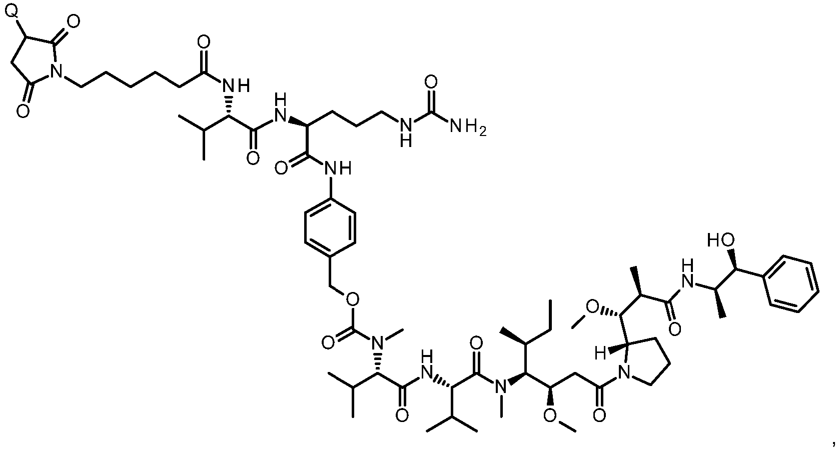 or a pharmaceutically acceptable salt or solvate thereof.

In some embodiments, the ADC has the structure: 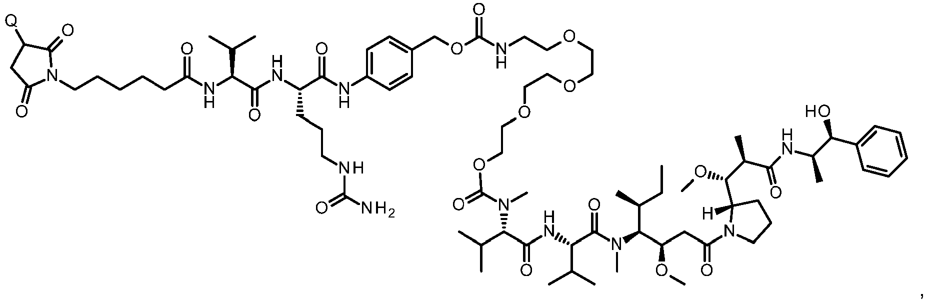 or a pharmaceutically acceptable salt or solvate thereof.

### DAR

The drug-antibody ratio (DAR) is the number of payloads (MMAE) on each ADC. The DAR will depend on the number of payloads per linker-payload moiety, and on the number of linker-payload moieties conjugated per antibody. The DAR may be dependent on the conditions for conjugation of the linker-payload moiety to the antibody.

In some embodiments of the present invention, the DAR may be from about 3 to 14, preferably from about 3 to 13, preferably from about 3 to 10, and most preferably from about 3 to 5. In some embodiments, the DAR is from about 3 to 13, from about 3 to 10, from about 3 to 6, from about 3 to 5, from about 3 to 4, from about 4 to 5, from about 5 to 10, from about 6 to 10, form about 7 to 9, from about 7 to 8, or from about 8 to 9.

In some embodiments, the DAR may be about 14 or less, about 13 or less, about 12 or less, about 11 or less, about 10 or less, about 9 or less, about 8 or less, about 7 or less, about 6 or less, about 5 or less, about 4 or less. In some embodiments, the DAR may be about 9 or less. In some embodiments, the DAR may be about 8 or less. In some embodiments, the DAR may be about 5 or less. In some embodiments, the DAR may be about 4 or less.

In some embodiments, the DAR may be about 4 or above, about 5 or above, about 6 or above, about 7 or above, about 8 or above, about 9 or above, about 10 or above, about 11 or above, about 12 or above, about 13 or above, about 14 or above. In some embodiments, the DAR may be about 3 or above. In some embodiments, the DAR may be about 4 or above. In some embodiments, the DAR may be about 7 or above. In some embodiments, the DAR may be about 8 or above.

In some embodiments, the DAR is from about 3 to 5.

In some embodiments, the DAR is from about 7 to 9.

In some embodiments, the DAR is from about 10 to 14.

In some embodiments, the DAR is about 3, about 4, about 5, about 6, about 7, about 8, or about 9. In some embodiments, the DAR is about 4 or about 8. In some embodiments the DAR is about 4 (for example, about 3.75). In some embodiments, the DAR is about 8.

### Conjuqation of linker-payload and antibody

Conjugation of the linker-payload unit and the antibody can be performed according to any suitable method known in the art. A representative method is described in the Examples below using vc-PABA-MMAE and anti-Endoglin antibody.

The features disclosed in the foregoing description, or in the following claims, or in the accompanying drawings, expressed in their specific forms or in terms of a means for performing the disclosed function, or a method or process for obtaining the disclosed results, as appropriate, may, separately, or in any combination of such features, be utilised for realising the invention in diverse forms thereof.

While the invention has been described in conjunction with the exemplary embodiments described above, many equivalent modifications and variations will be apparent to those skilled in the art when given this disclosure. Accordingly, the exemplary embodiments of the invention set forth above are considered to be illustrative and not limiting. Various changes to the described embodiments may be made without departing from the spirit and scope of the invention.

For the avoidance of any doubt, any theoretical explanations provided herein are provided for the purposes of improving the understanding of a reader. The inventors do not wish to be bound by any of these theoretical explanations.

Any section headings used herein are for organizational purposes only and are not to be construed as limiting the subject matter described.

Throughout this specification, including the claims which follow, unless the context requires otherwise, the word "comprise" and "include", and variations such as "comprises", "comprising", and "including" will be understood to imply the inclusion of a stated integer or step or group of integers or steps but not the exclusion of any other integer or step or group of integers or steps.

It must be noted that, as used in the specification and the appended claims, the singular forms "a," "an," and "the" include plural referents unless the context clearly dictates otherwise. Ranges may be expressed herein as from "about" one particular value, and/or to "about" another particular value. When such a range is expressed, another embodiment includes from the one particular value and/or to the other particular value. Similarly, when values are expressed as approximations, by the use of the antecedent "about," it will be understood that the particular value forms another embodiment. The term "about" in relation to a numerical value is optional and means for example +/- 10%.

### Examples

Structures of some specific conjugates which are referred to herein are shown in Table 1. OMTX103 is according to the present invention. OMTX603-2 and OMTX703 are included as comparative examples.

**Table 1. Conjugates referred to herein**

| **Name** | **DAR** | **Structure** |
|---|---|---|
| OMTX103 | 3.75 | 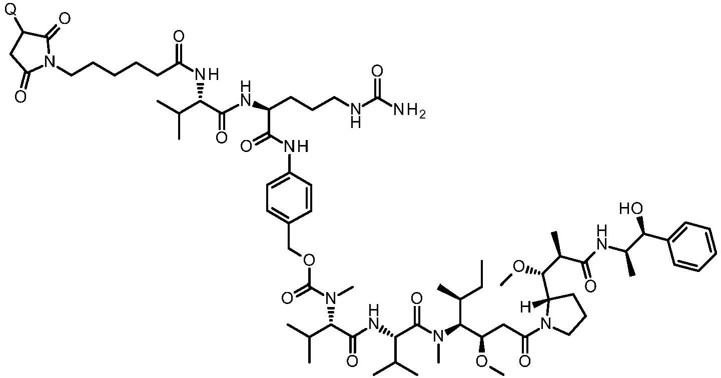 |
| OMTX603-2 (comparative) | 4.15 | 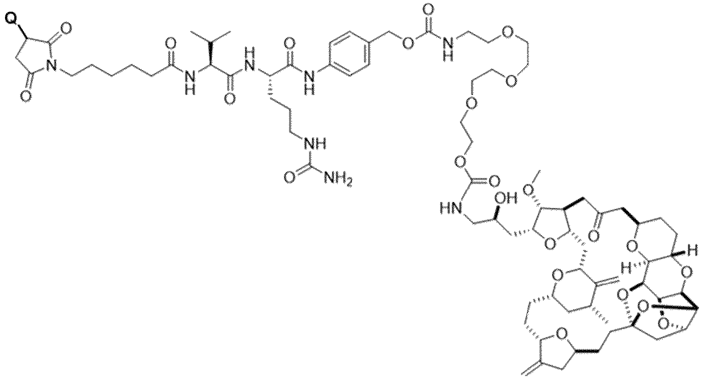 |
| OMTX703 (comparative) | 3.8 | 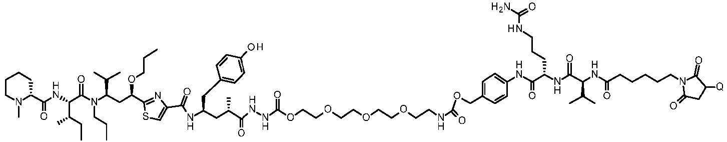 |

### EXAMPLE 1: Production of anti-ENG ADCs

An anti-endoglin ADC was generated, comprising an anti-endoglin antibody (OMTX003) and MMAE as a payload. The ADC was termed OMTX103.

OMTX003 was specific for the extracellular region of human ENG (amino acids 26-561 of SEQ ID NO: 25) and may otherwise be referred to as A5-IgG1. The amino acid sequences of anti-human ENG IgG1 A5 (A5-IgG1) heavy chain (HC) and light chain (LC), respectively are shown below:

### Anti-human Endoglin A5-IgG1-HC:

METDTLLLWVLLLWVPGSTG
EVQLLESGGGLVQPGGSLRLSCAASGFTFSSYAMSWVRQAPGKGLEWVSAIYGSDGDTTYADSVKGRF
TISRDNSKNTLYLQMNSLRAEDTAVYYCARVFYTAGFDYWGQGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKP
SNTKVDKKVEPKSCDKTHTCPPCPAPPVAGPSVFLFPPKPKDTLMISRTPEVTCVVVDVSHEDPEVKFN
WYVDGVEVHNAKTKPREEQYNSTYRVVSVLTVLHQDWLNGKEYKCKVSNKGLPSSIEKTISKAKGQPRE
PQVYTLPPSRDELTKNQVSLTCLVKGFYPSDIAVEWESNGQPENNYKTTPPVLDSDGSFFLYSKLTVDKS
RWQQGNVFSCSVMHEALHNHYTQKSLSLSPGK (SEQ ID NO: 1)

| | |
|---|---|
| *aa* | *447* |
| *MW of processed HC* | *48,703* |
| *Theoretical pl* | *8.36* |
| *Potential glycosylation site (double underlined):* | *N297* |

*Mutations leading to ADCC and CDC deficiency are shown in bold italics (see also* WO 99/58572) *Signal sequence is shown boxed*
*VH domain is underlined; CDRH1-H3 are shown in bold and curved underlined.*

### Anti-human Endoglin A5-IgG1-LC:

METETDTLLLWVLLLWVPGSTG
DIELTQSPSSLSASVGDRVTITCRASQSISSSLNWYQQKPGKAPKLLIYAASSLQSGVPSRFSGSGSGTD
FTLTISSLQPEDFATYYCQQAPAKPPTFGQGTKLEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFN
RGEC (SEQ ID NO: 2)

| | |
|---|---|
| *aa* | *214* |
| *MW of processed HC* | *23,113* |
| *theoretical pl* | *7.76* |

*signal sequence is boxed*
*VL domain is underlined; CDRL1-L3 are shown in bold and curved underlined.*

A5-IgG1-HC - without signal sequence: 
EVQLLESGGGLVQPGGSLRLSCAASGFTFSSYAMSWVRQAPGKGLEWVSAIYGSDGDTTYADSVKGRF
TISRDNSKNTLYLQMNSLRAEDTAVYYCARVFYTAGFDYWGQGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKP
SNTKVDKKVEPKSCDKTHTCPPCPAPPVAGPSVFLFPPKPKDTLMISRTPEVTCVVVDVSHEDPEVKFN
WYVDGVEVHNAKTKPREEQYNSTYRVVSVLTVLHQDWLNGKEYKCKVSNKGLPSSIEKTISKAKGQPRE
 
PQVYTLPPSRDELTKNQVSLTCLVKGFYPSDIAVEWESNGQPENNYKTTPPVLDSDGSFFLYSKLTVDKS
RWQQGNVFSCSVMHEALHNHYTQKSLSLSPGK (SEQ ID NO: 3)
A5-lgG1-LC - without signal sequence:

DIELTQSPSSLSASVGDRVTITCRASQSISSSLNWYQQKPGKAPKLLIYAASSLQSGVPSRFSGSGSGTD
FTLTISSLQPEDFATYYCQQAPAKPPTFGQGTKLEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFN
RGEC (SEQ ID NO: 4)

### Linker-payload for OMTX103 (vc-PABA-MMAE)

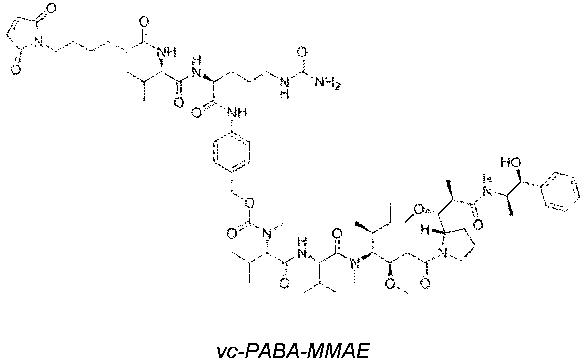

vc-PABA-MMAE is commercially available from suppliers such as MedChemExpress. It has CAS number 646502-53-6. Its IUPAC name is [4-[[5-(carbamoylamino)-2-[[2-[6-(2,5-dioxopyrrol-1-yl)hexanoylamino]-3-methylbutanoyl]amino]pentanoyl]amino]phenyl]methyl N-[1-[[1-[[1-[2-[3-[(1-hydroxy-1-phenylpropan-2-yl)amino]-1-methoxy-2-methyl-3-oxopropyl]pyrrolidin-1-yl]-3-methoxy-5-methyl-1-oxoheptan-4-yl]-methylamino]-3-methyl-1-oxobutan-2-yl]amino]-3-methyl-1-oxobutan-2-yl]-N-methylcarbamate. Its preparation is described in WO 2004/010957 A2. The valine-citruline (vc) unit is a lysosomally cleavable dipeptide.

The vc-PABA-MMAE linker-payload moiety conjugates to the antibody via the maleimide group, as shown below:

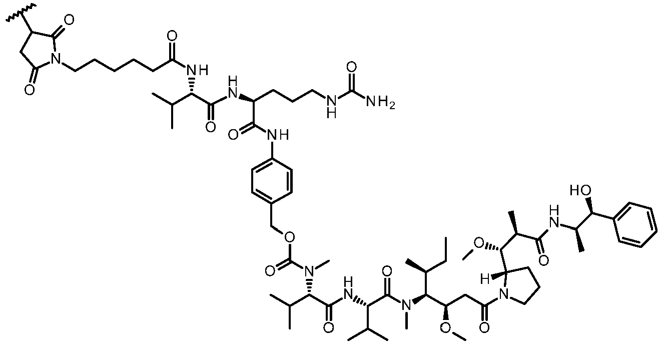

### Conjugation of vc-PABA-MMAE conjugation to anti-Endoglin antibody:

Equivalents (eq) are with reference to the antibody. The anti-Endoglin antibody, is placed in a conjugation buffer made of 10-50 mM NaPi or sodium phosphate or histidine or sodium succinate, 100-200 mM NaCl, 100-200 mM KCI, 2-20 mM EDTA, pH 7.0-7.5 (e.g. at an antibody concentration of about 5 mg/mL, such as 5.0 mg/mL), and placed in a glass reaction bottle. 1.8-3.6 eq (e.g. 2.15 eq) TCEP is added thereto to conduct reduction reaction of the antibody. The reduction reaction is conducted in the reaction solution at 18-25 °C (e.g. ~22 °C) for 1.5-24 hours (e.g. ~16 hours), and the estimated DAR may be calculated by hydrophobic interaction chromatography (HIC)-HPLC. After the reduction reaction is complete, 5-15% DMSO or DMA or ACN and 4.0-9.0 eq (e.g. 8 eq) mc-PABA-MMAE are added. After 0.5-2 hours (e.g. 1 hour) of reaction, a sample is taken to confirm the DAR value. Afterwards, a buffer solution including 10-50 mM succinic acid or sodium citrate or sodium acetate or/and arginine, pH 4.5-6.0, is added to dilute the reaction solution 3-6 times and to terminate the conjugation reaction.

The diluted mc-PABA-MMAE ADC mixture is filtered by 0.2 µm filter, followed by diafiltration concentration and purification to a buffer solution including 10-50 mM succinic acid or sodium citrate or sodium acetate or/and arginine, pH 4.5-6.0, with 30-100 kDa filter membrane (e.g., a 15 mL, 30 kDa Amicon^{®} ultrafiltration membrane) to remove mc-PABA-MMAE, impurities related to mc-PABA-MMAE and residual solvent, e.g., DMSO, DMA or ACN. After the concentration is adjusted to 10-30 mg/mL (e.g. to about 12 mg/mL, such as 12.13 mg/mL), excipient (50-65% sucrose, and 0.02-0.4% tween 20 or tween 80) is added and the mixture is filtered by 0.2 µm filter to give mc-PABA-MMAE ADC DS. Finally, a sample is taken to confirm the concentration, the DAR value (HIC-HPLC or Reduced RPLC) and HMW (%) (High Molecular Weight species). The product is stored under -20 to -80°C with a yield of 81-90% (e.g. 89.77%).

The average DAR of the representative batch mc-PABA-MMAE ADC (OMTX103) was 3.75 (measured using HIC-HPLC) with purity of 99.35% and HMW of 0.65% (measured using SEC-HPLC).

### EXAMPLE 2: In vitro characterization; binding and cytotoxicity

An anti-human Endoglin ADC was generated conjugating OMTX003 anti-huENG antibody to MMAE microtubule inhibitor (mc-PABA-MMAE) to form ADC OMTX103.

Cell binding of OMTX103 was analyzed via flow cytometry using endoglin-expressing HT1080-WT cells. HT-1080 human cells are epithelial cells derived from connective tissue from a patient with Fibrosarcoma that can be used in research and assay development. Relative mean fluorescence intensity (MFI) was measured to assess binding. OMTX703, an ADC with anti-Endoglin antibody conjugated to a cytolysin payload (see Table 1), was also included in the experiment to compare both payloads. OMTX703 is disclosed in WO 2015/118031 and also comprises OMTX003, but with a different linker and payload to the ADC of the present invention.

The relative MFI is calculated as follows: $relative MFI = \frac{\left({value}_{sample}-\left({value}_{detection antibody}-{value}_{cells}\right)\right)}{{value}_{cells}}$

Where: valueₛₐₘₚₗₑ is obtained by testing the titration of the antibodies; value_{detection antibody} is obtained by testing the cells only in the presence of the detection antibody; and value_{cells} is obtained by testing the cells on their own (without adding any agent).

MFI values were obtained by flow cytometry analysis using a MACSQuant^{®} analyzer from Miltenyi. For the detection of cell-bound antibodies (MFI_{construct}), one detection antibody (anti-human-Fc-PE for the detection of human IgG molecules) was used in the flow cytometry analysis. As a control, the detection antibody was also tested with cells (MFI_{detection control}), and the MFI was also calculated for cells without adding any antibody (MFI_{cells}).

All conjugates showed a concentration-dependent binding to HT1080-WT cells in the sub-nanomolar range (Figure 1A), with EC50 values of 236.5 pM for OMTX103 and 110 pM for OMTX703. (Table 2).

The different ADCs (OMTX103 and OMTX703) were evaluated for their *in vitro* cytotoxic activity via cell viability assay using HT1080-WT cells. OMTX103 and both batches of OMTX703 ADCs showed a concentration-dependent killing of these cells incubated for 3 days. A higher cytotoxic activity of the MMAE conjugate was found compared to the cytolysin one, with an IC50 value of 95.5 pM for OMTX103 and 7,400 pM for OMTX703 (Figure 1B). Hence, OMTX103 unexpectedly displays significantly improved cytotoxic activity compared to existing anti-Endoglin ADCs.

**Table 2: Overview of anti-endoglin ADC molecules concerning binding through flow cytometry (FC) and induction of cytotoxicity using cell viability assay (incubated for 72h).**

| | | |
|---|---|---|
| Values are mean averages; n=1 | | |

| | **EC50 (FC - HT1080-WT) [pM]** | **IC50 (Cytotox - HT1080-WT) [pM]** |
|---|---|---|
| **OMTX103** | 236.5 | 95.5 |
| **OMTX703** | 110 | 7400 |

### EXAMPLE 3: In vivo antitumoral efficacy

OMTX103 in vivo anti-tumoral efficacy was tested in a gastric cancer PDX model, ST-02-0318, with high expression of endoglin.

OMTX103 was administrated at two different weekly i.v doses, 10 and 20mg/kg. After 4 weeks of treatment, OMTX103 showed a dose-dependent anti-tumoral effect, with a tumor growth inhibition (TGI) of 37.26% at 5 mg/kg and a TGI of 105.89% at 20 mg/kg, showing a marked decrease in tumor volume when treated with OMTX103 compared to the vehicle, with complete tumor regression up to Day 45 and no regrowth 24 days after the last dose (Figure 2A). In contrast, the anti-tumoral effect of comparative ADC, OMTX603-2 (see structure in Table 1), was less significant, particularly in the 20 mg/kg dose, with a TGI of 34.72% at 5 mg/kg, and a TGI of 72.83% at 20 mg/kg. Hence, OMTX103 unexpectedly displays significantly improved anti-tumor properties compared to other anti-Endoglin ADCs.

TGI was calculated according to the following formula: TGI (%) = [1 - (T*ᵢ* - T₀)/ (V*ᵢ* - V₀)] × 100, where Tᵢ is the average tumor volume of a treatment group on a given day, T₀ is the average tumor volume of the treatment group on the day of the start of treatment, Vᵢ is the average tumor volume of the vehicle control group on the same day as Tᵢ, and V₀ is the average tumor volume of the vehicle group at the start of treatment.

No OMTX103 tolerability issues were observed when monitoring animal weight (Figure 2B).

### Sequences

***Anti-human Endoglin A5-IgG1-HC:*** 
METDTLLLWVLLLWVPGSTG
EVQLLESGGGLVQPGGSLRLSCAASGFTFSSYAMSWVRQAPGKGLEWVSAIYGSDGDTTYADSVKGRF
TISRDNSKNTLYLQMNSLRAEDTAVYYCARVFYTAGFDYWGQGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKP
SNTKVDKKVEPKSCDKTHTCPPCPAPPVAGPSVFLFPPKPKDTLMISRTPEVTCVVVDVSHEDPEVKFN
WYVDGVEVHNAKTKPREEQYNSTYRVVSVLTVLHQDWLNGKEYKCKVSNKGLPSSIEKTISKAKGQPRE
PQVYTLPPSRDELTKNQVSLTCLVKGFYPSDIAVEWESNGQPENNYKTTPPVLDSDGSFFLYSKLTVDKS
RWQQGNVFSCSVMHEALHNHYTQKSLSLSPGK (SEQ ID NO: 1)
***Anti-human Endoglin A5-IgG1-LC:*** 
METDTLLLWVLLLWVPGSTG
DIELTQSPSSLSASVGDRVTITCRASQSISSSLNWYQQKPGKAPKLLIYAASSLQSGVPSRFSGSGSGTD
FTLTISSLQPEDFATYYCQQAPAKPPTFGQGTKLEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFN
RGEC (SEQ ID NO: 2)
A5-IgG1-HC - without signal sequence: 
EVQLLESGGGLVQPGGSLRLSCAASGFTFSSYAMSWVRQAPGKGLEWVSAIYGSDGDTTYADSVKGRF
TISRDNSKNTLYLQMNSLRAEDTAVYYCARVFYTAGFDYWGQGTLVTVSSASTKGPSVFPLAPSSKSTS
 
GGTAALGCLVKDYFPEPVTVSWNSGALTSGVHTFPAVLQSSGLYSLSSVVTVPSSSLGTQTYICNVNHKP
SNTKVDKKVEPKSCDKTHTCPPCPAPPVAGPSVFLFPPKPKDTLMISRTPEVTCVVVDVSHEDPEVKFN
WYVDGVEVHNAKTKPREEQYNSTYRVVSVLTVLHQDWLNGKEYKCKVSNKGLPSSIEKTISKAKGQPRE
PQVYTLPPSRDELTKNQVSLTCLVKGFYPSDIAVEWESNGQPENNYKTTPPVLDSDGSFFLYSKLTVDKS
RWQQGNVFSCSVMHEALHNHYTQKSLSLSPGK (SEQ ID NO: 3)
A5-lgG1-LC - without signal sequence: 
DIELTQSPSSLSASVGDRVTITCRASQSISSSLNWYQQKPGKAPKLLIYAASSLQSGVPSRFSGSGSGTD
FTLTISSLQPEDFATYYCQQAPAKPPTFGQGTKLEIKRTVAAPSVFIFPPSDEQLKSGTASVVCLLNNFYP
REAKVQWKVDNALQSGNSQESVTEQDSKDSTYSLSSTLTLSKADYEKHKVYACEVTHQGLSSPVTKSFN
RGEC (SEQ ID NO: 4)
A5-VH: 
EVQLLESGGGLVQPGGSLRLSCAASGFTFSSYAMSWVRQAPGKGLEWVSAIYGSDGDTTYADSVKGRF
TISRDNSKNTLYLQMNSLRAEDTAVYYCARVFYTAGFDYWGQGTLVTVSS (SEQ ID NO: 5)
A5-VL: 
DIELTQSPSSLSASVGDRVTITCRASQSISSSLNWYQQKPGKAPKLLIYAASSLQSGVPSRFSGSGSGTD
FTLTISSLQPEDFATYYCQQAPAKPPTFGQGTKLEIKR (SEQ ID NO: 6)
A5-CDRH1:
   SYAMS (SEQ ID NO: 7)
A5-CDRH2:
   AIYGSDGDTTY (SEQ ID NO: 8)
A5-CDRH3:
   VFYTAGFDY (SEQ ID NO: 9)
A5-CDRL1:
   RASQSISSSLN (SEQ ID NO: 10)
A5-CDRL2:
   AASSLQS (SEQ ID NO: 11)
A5-CDRL3:
   QQAPAKPPT (SEQ ID NO: 12)
***Anti-murine Endoglin mE12-IgG1-LC:*** 
METDTLLLWVLLLWVPGSTG
SSELIQDPAVSVALGQTVRITCQGDSLRSYYASWYQQKPGQAPVLVIYGKNNRPSGIPDRFSGSSSGNT
ASLTITGAQAEDEADYYCNSRDSSGTVFGGGTKLTVLGQPKANPTVTLFPPSSEELQANKATLVCLISDFY
PGAVTVAWKADGSPVKAGVETTKPSKQSNNKYAASSYLSLTPEQWKSHRSYSCQVTHEGSTVEKTVAP
TECS (SEQ ID NO: 14)
mE12-IgG1-HC - without signal sequence: 
EVQLVESGGGVVQPGRSLRLSCAASGFTFSSYGMHWVRQAPGKGLVWVSRINSDGSSTSYADSVKGR
FTISRDNSKNTLYLQMNSLRAEDTAVYYCARATGTWVMSWGQGTLVTVSSASTKGPSVFPLAPSSKSTS
GGTAALGCLVKDYFPEPVTVSWNSGALTSGVHTFPAVLQSSGLYSLSSVVTVPSSSLGTQTYICNVNHKP
SNTKVDKKVEPKSCDKTHTCPPCPAPPVAGPSVFLFPPKPKDTLMISRTPEVTCVVVDVSHEDPEVKFN
WYVDGVEVHNAKTKPREEQYNSTYRVVSVLTVLHQDWLNGKEYKCKVSNKGLPSSIEKTISKAKGQPRE
PQVYTLPPSRDELTKNQVSLTCLVKGFYPSDIAVEWESNGQPENNYKTTPPVLDSDGSFFLYSKLTVDKS
RWQQGNVFSCSVMHEALHNHYTQKSLSLSPGK (SEQ ID NO: 15)
mE12-IgG1-LC - without signal sequence: 
SSELIQDPAVSVALGQTVRITCQGDSLRSYYASWYQQKPGQAPVLVIYGKNNRPSGIPDRFSGSSSGNT
ASLTITGAQAEDEADYYCNSRDSSGTVFGGGTKLTVLGQPKANPTVTLFPPSSEELQANKATLVCLISDFY
PGAVTVAWKADGSPVKAGVETTKPSKQSNNKYAASSYLSLTPEQWKSHRSYSCQVTHEGSTVEKTVAP
TECS (SEQ ID NO: 16)
mE12-VH: 
EVQLVESGGGVVQPGRSLRLSCAASGFTFSSYGMHWVRQAPGKGLVWVSRINSDGSSTSYADSVKGR
FTISRDNSKNTLYLQMNSLRAEDTAVYYCARATGTWVMSWGQGTLVTVSS (SEQ ID NO: 17)
mE12-VL: 
SSELIQDPAVSVALGQTVRITCQGDSLRSYYASWYQQKPGQAPVLVIYGKNNRPSGIPDRFSGSSSGNT
ASLTITGAQAEDEADYYCNSRDSSGTVFGGGTKLTVLG (SEQ ID NO: 18)
mE12-CDRH1:
   SYGMH (SEQ ID NO: 19)
mE12-CDRH2:
   RINSDGSSTSYADSVKG (SEQ ID NO: 20)
mE12-CDRH3:
   ATGTWVMS (SEQ ID NO: 21)
mE12-CORL1:
   QGDSLRSYYAS (SEQ ID NO: 22)
mE12-CDRL2:
   GKNNRPS (SEQ ID NO: 23)
mE12-CDRL3:
   NSRDSSGTV (SEQ ID NO: 24)
Human endoglin 
MDRGTLPLAVALLLASCSLSPTSLAETVHCDLQPVGPERGEVTYTTSQVSKGCVAQAPNA
ILEVHVLFLEFPTGPSQLELTLQASKQNGTWPREVLLVLSVNSSVFLHLQALGIPLHLAY
NSSLVTFQEPPGVNTTELPSFPKTQILEWAAERGPITSAAELNDPQSILLRLGQAQGSLS
FCMLEASQDMGRTLEWRPRTPALVRGCHLEGVAGHKEAHILRVLPGHSAGPRTVTVKVEL
SCAPGDLDAVLILQGPPYVSWLIDANHNMQIWTTGEYSFKIFPEKNIRGFKLPDTPQGLL
GEARMLNASIVASFVELPLASIVSLHASSCGGRLQTSPAPIQTTPPKDTCSPELLMSLIQ
TKCADDAMTLVLKKELVAHLKCTITGLTFWDPSCEAEDRGDKFVLRSAYSSCGMQVSASM
ISNEAVVNILSSSSPQRKKVHCLNMDSLSFQLGLYLSPHFLQASNTIEPGQQSFVQVRVS
PSVSEFLLQLDSCHLDLGPEGGTVELIQGRAAKGNCVSLLSPSPEGDPRFSFLLHFYTVP
IPKTGTLSCTVALRPKTGSQDQEVHRTVFMRLNIISPDLSGCTSKGLVLPAVLGITFGAF
LIGALLTAALWYIYSHTRSPSKREPVVAVAAPASSESSSTNHSIGSTQSTPCSTSS (SEQ ID NO: 25)
Murine endoglin

MDRGVLPLPITLLFVIYSFVPTTGLAERVGCDLQPVDPTRGEVTFTTSQVSEGCVAQAAN
AVREVHVLFLDFPGMLSHLELTLQASKQNGTETQEVFLVLVSNKNVFVKFQAPEIPLHLA
YDSSLVIFQGQPRVNITVLPSLTSRKQILDWAATKGAITSIAALDDPQSIVLQLGQDPKA
PFLCLPEAHKDMGATLEWQPRAQTPVQSCRLEGVSGHKEAYILRILPGSEAGPRTVTVMM
ELSCTSGDAILILHGPPYVSWFIDINHSMQILTTGEYSVKIFPGSKVKGVELPDTPQGLI
AEARKLNASIVTSFVELPLVSNVSLRASSCGGVFQTTPAPVVTTPPKDTCSPVLLMSLIQ
PKCGNQVMTLALNKKHVQTLQCTITGLTFWDSSCQAEDTDDHLVLSSAYSSCGMKVTAHV
VSNEVIISFPSGSPPLRKKVQCIDMDSLSFQLGLYLSPHFLQASNTIELGQQAFVQVSVS
PLTSEVTVQLDSCHLDLGPEGDMVELIQSRTAKGSCVTLLSPSPEGDPRFSFLLRVYMVP
TPTAGTLSCNLALRPSTLSQEVYKTVSMRLNIVSPDLSGKGLVLPSVLGITFGAFLIGAL
LTAALWYIYSHTRGPSKREPVVAVAAPASSESSSTNHSIGSTQSTPCSTSSMA (SEQ ID NO: 26)

### References

A number of publications are cited above in order to more fully describe and disclose the invention and the state of the art to which the invention pertains. Full citations for these references are provided below. The entirety of each of these references is incorporated herein.

For standard molecular biology techniques, see Sambrook, J., Russel, D.W. Molecular Cloning, A Laboratory Manual. 3 ed. 2001, Cold Spring Harbor, New York: Cold Spring Harbor Laboratory Press
Shastry, Mythili et al. "Rise of Antibody-Drug Conjugates: The Present and Future." American Society of Clinical Oncology educational book. American Society of Clinical Oncology. Annual Meeting vol. 43 (2023): e390094. doi:10.1200/EDBK_390094
Fu, Zhiwen et al. "Antibody drug conjugate: the "biological missile" for targeted cancer therapy." Signal transduction and targeted therapy vol. 7,1 93. 22 Mar. 2022, doi:10.1038/s41392-022-00947-7
Pettinato MC. Introduction to Antibody-Drug Conjugates. Antibodies (Basel). 2021 Oct 27;10(4):42. doi: 10.3390/antib10040042. PMID: 34842621; PMCID: PMC8628511.
Marei, Hany E. et al. "Potential of antibody-drug conjugates (ADCs) for cancer therapy." Cancer Cell International vol. 22, 255 (2022). doi:10.1186/s12935-022-02679-8
Wang, Zhijia et al. "Antibody-drug conjugates: Recent advances in payloads." Acta pharmaceutica Sinica. B vol. 13,10 (2023): 4025-4059. doi:10.1016/j.apsb.2023.06.015
Samantasinghar, Anupama et al. "A comprehensive review of key factors affecting the efficacy of antibody drug conjugate." Biomedicine & pharmacotherapy = Biomedecine & pharmacotherapie vol. 161 (2023): 114408. Doi:10.1016/j.biopha.2023.114408
Riccardi, Federico et al. "A comprehensive overview on antibody-drug conjugates: from the conceptualization to cancer therapy." Frontiers in pharmacology vol. 14 1274088. 18 Sep. 2023, doi:1 0.3389/fphar.2023.127 4088

## Claims

1. An antibody-drug conjugate having a structure of Formula (I), or a pharmaceutically acceptable salt or solvate thereof: 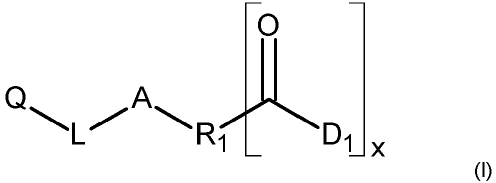 wherein:
Q is an antibody that selectively binds Endoglin;
L is a linker;
A is a bond or 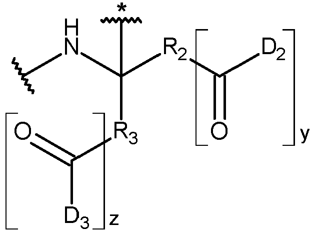 where the asterisk (*) marks the point of attachment to R₁;
each of D₁, D₂ and D₃ are independently MMAE;
each of x, y and z is independently 0 or 1, with a provision that at least one of x, y, and z is 1;
each of R₁, R₂, and R₃ is independently selected from: a bond, hydrogen, 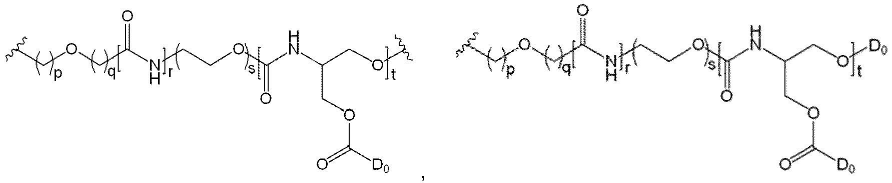 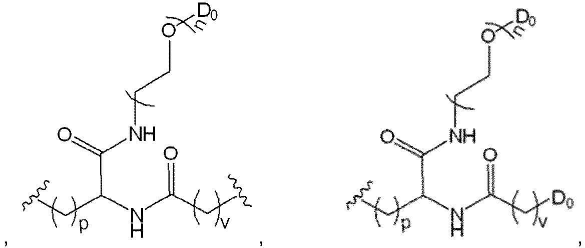 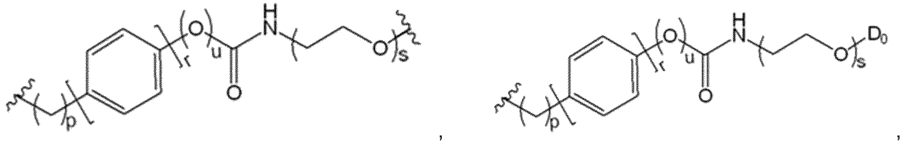 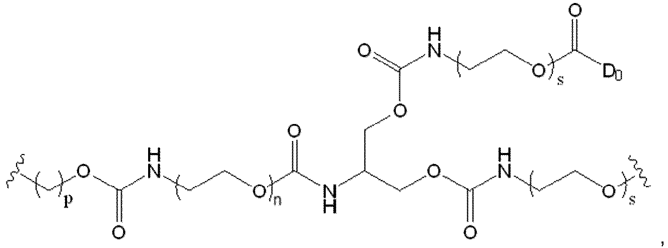 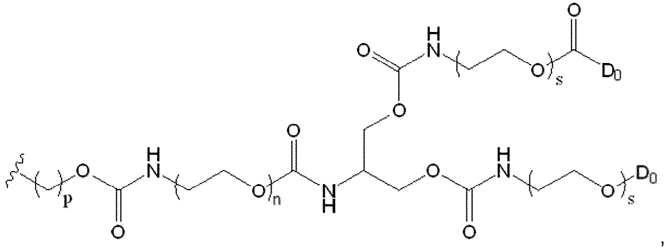 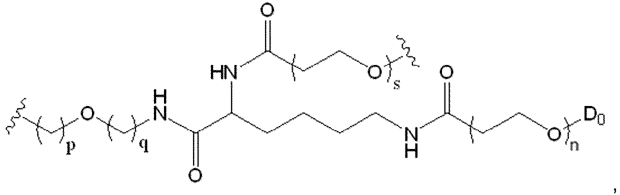 and 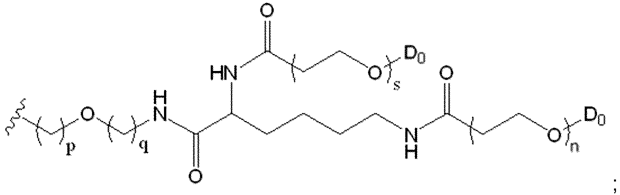
wherein:
p is 1, 2, or 3;
q is 0, 1, or 2;
r is 0 or 1;
s is an integer from 0 to 37;
t is 0 or 1;
u is 0 or 1;
v is 1, 2, or 3; and
n is an integer from 1 to 37; or
x and y are 0, and R₁ and R₂ together form =O and R₃ is 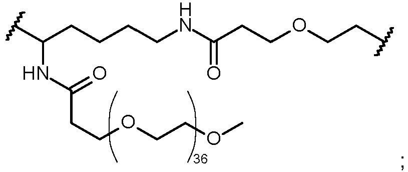
D₀ is methyl, propargyl, or MMAE;
with a provision that at least one of R₁, R₂, and R₃ is not hydrogen;
if q, r, s, and t are 0, then x + y + z > 1;
if x is 0, then R₁ is selected from: hydrogen, 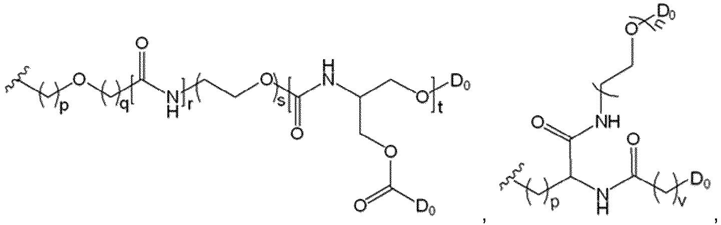 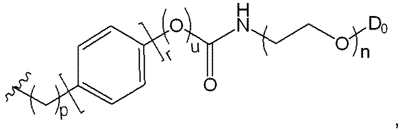 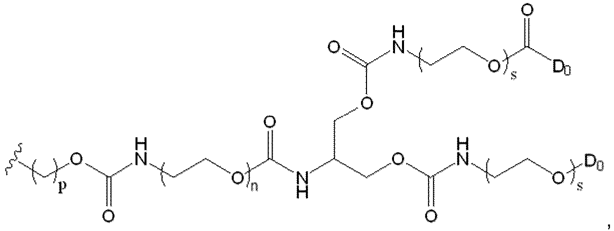 and 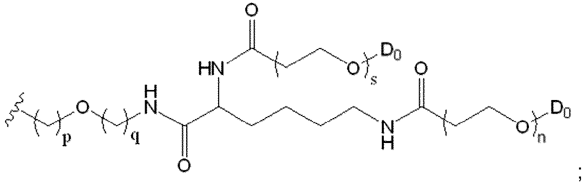
if y is 0, then R₂ is selected from: hydrogen, 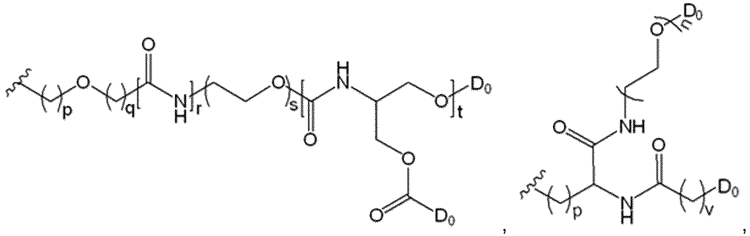 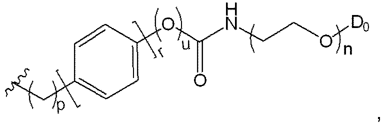 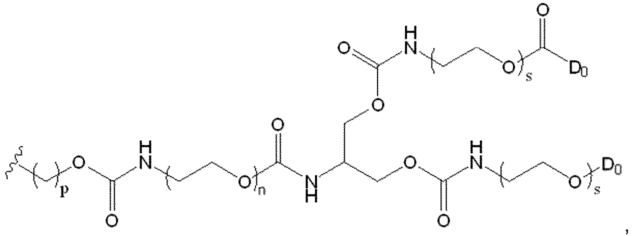 and 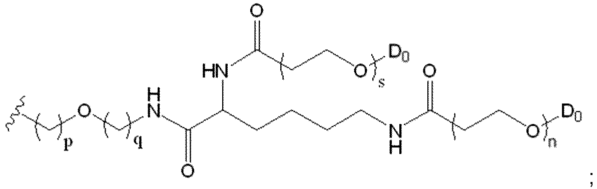
if z is 0, then R₃ is selected from: hydrogen, 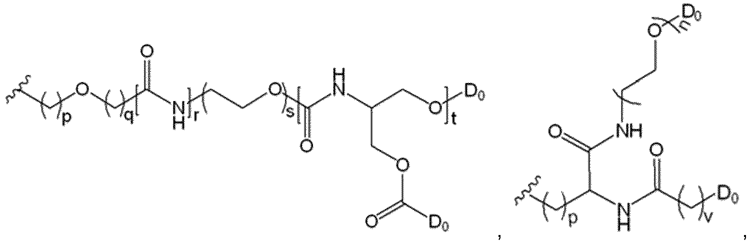 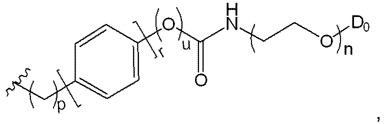 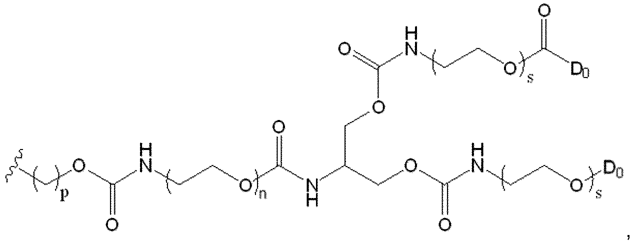 and 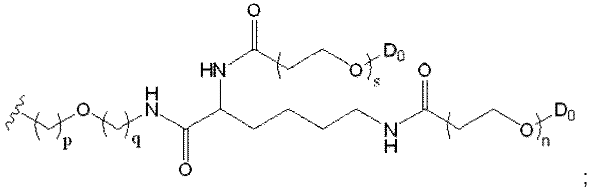
optionally, if x + y + z = 1, and each of R₁, R₂, and R₃ independently is 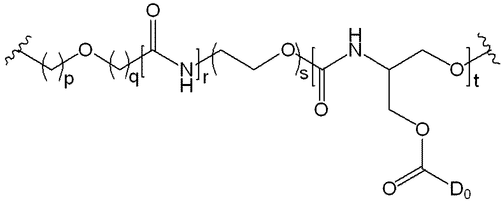 or 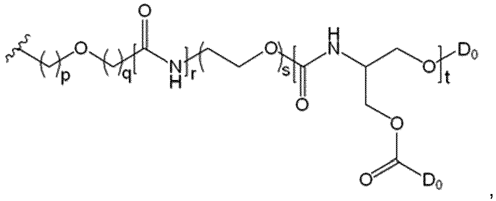 then p is not 1; and
optionally, if each of R₁, R₂, and R₃ independently is 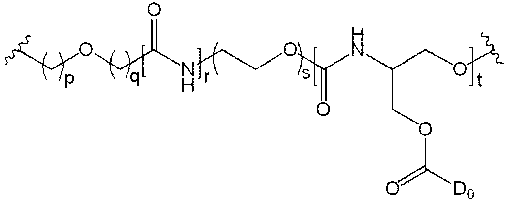 or 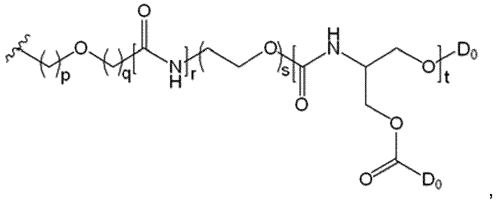 and r, s, and t are each 0, then x + y + z > 1.

2. The conjugate according to claim 1, having a structure according to Formula (II), or a pharmaceutically acceptable salt or solvate thereof: 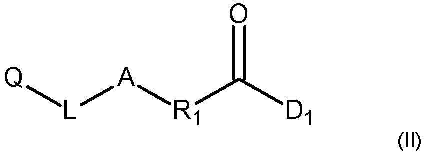 wherein:
Q is an antibody that selectively binds Endoglin;
L is a linker;
A is a bond or -NH-CH₂-;
D₁ is MMAE;
R₁ is selected from: a bond, 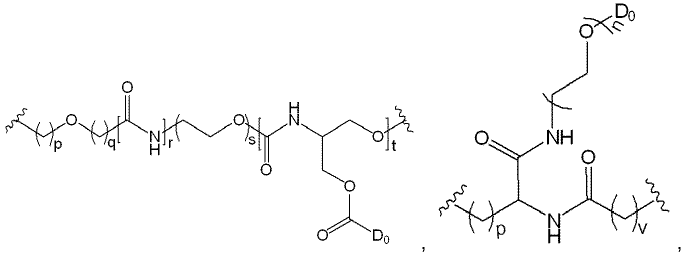 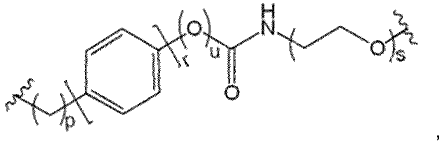 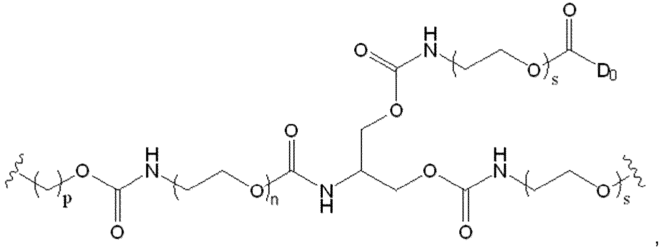 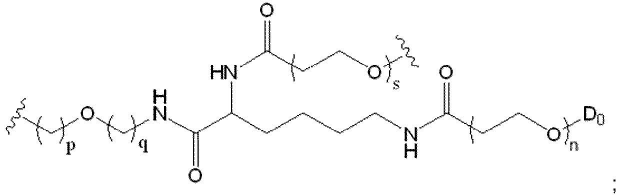
wherein:
p is 1, 2, or 3;
q is 0, 1, or 2;
r is 0 or 1;
s is an integer from 0 to 37;
t is 0 or 1;
u is 0 or 1;
v is 1, 2, or 3; and
n is an integer from 1 to 37; and
if R₁ is 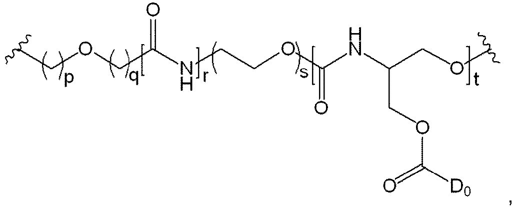 then p is not 1.

3. The conjugate according to claim 1 or 2, wherein L is selected from: 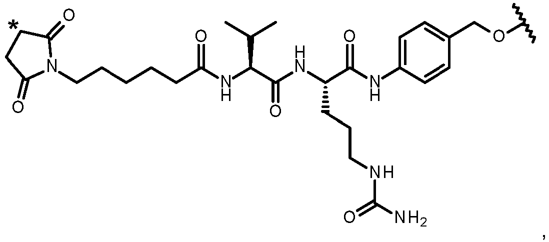 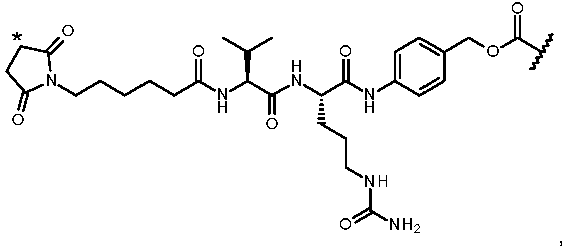 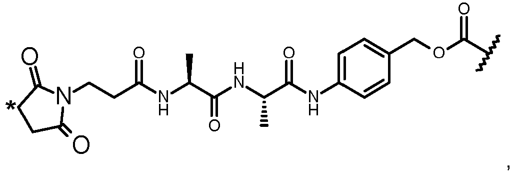 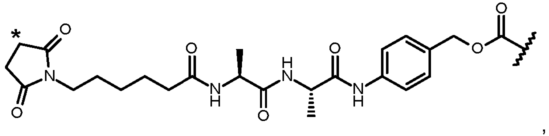 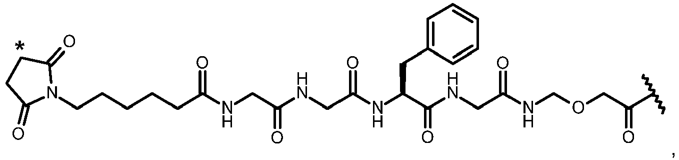 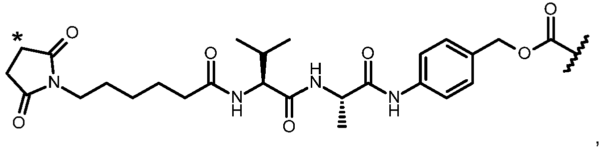 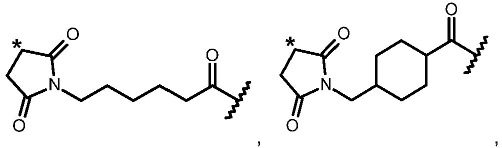 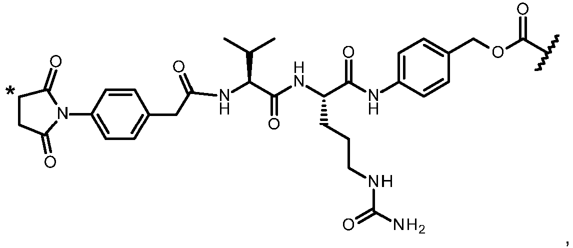 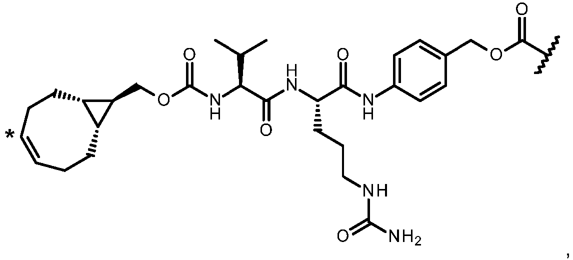 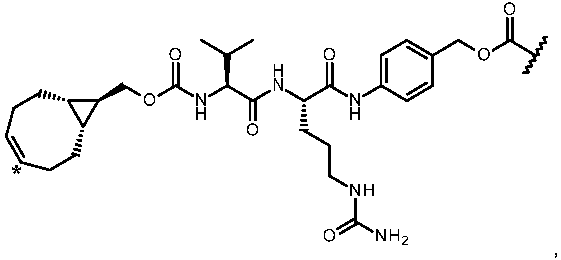 and 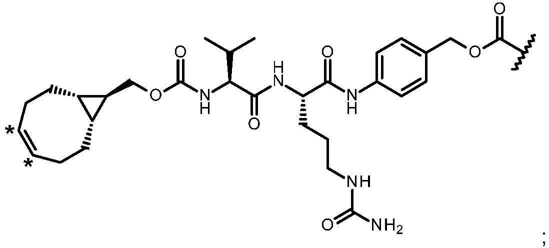 where the asterisk (*) marks the point of attachment to the antibody Q.

4. The conjugate according to any preceding claim, wherein L is:
(i) 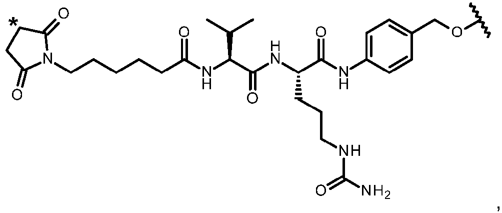 or
(ii) 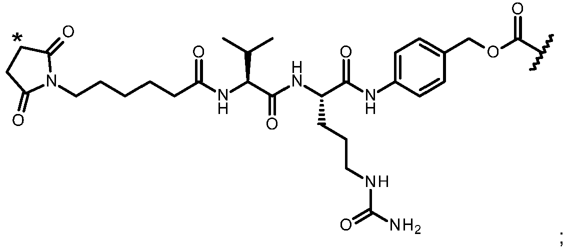
where the asterisk (*) marks the point of attachment to Q.

5. The conjugate according to any preceding claim, wherein R₁ is a bond or

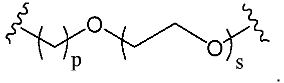

6. The conjugate according to any preceding claim, wherein:
L has structure
(i) 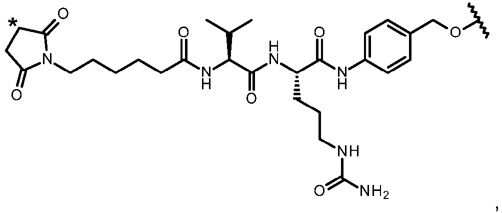
where the asterisk (*) marks the point of attachment to Q;
A is a bond; and
R₁ is a bond.

7. The conjugate according to any one of claims 1 to 5, wherein:
L has structure
(ii) 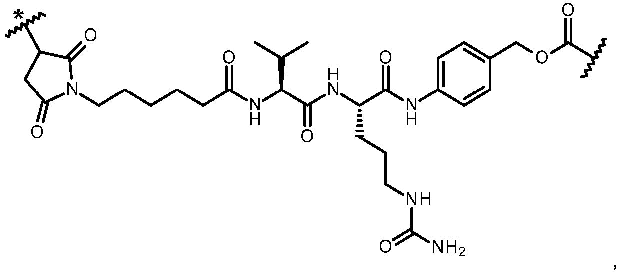
where the asterisk (*) marks the point of attachment to Q;
A is -NH-CH₂-; and
R₁ is 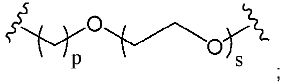
optionally wherein p is 1 and s is 3.

8. The conjugate according to any one of claims 1 to 5, wherein the conjugate is: 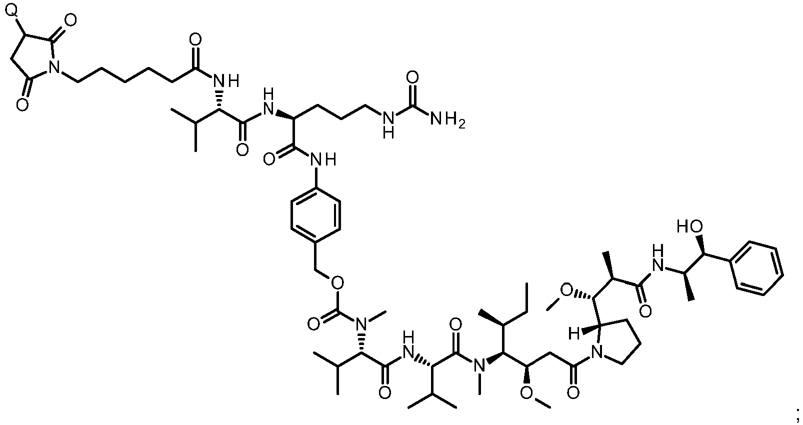 or 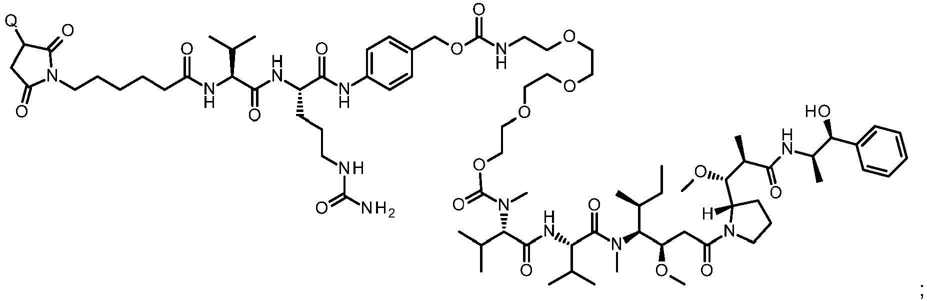 or a pharmaceutically acceptable salt or solvate thereof.

9. The conjugate according to any preceding claim, wherein the drug antibody ratio is from about 3 to about 13, preferably wherein the antibody drug ratio is from about 3 to about 10, preferably wherein the antibody drug ratio is from about 3 to about 5.

10. The conjugate according to claim 9, wherein the drug antibody ratio is from about 7 to about 9.

11. The conjugate according to any preceding claim, wherein the antibody is a monoclonal antibody or binding fragment thereof that selectively binds to an extracellular region of human Endoglin.

12. The conjugate according to claim 11, wherein the antibody comprises heavy chain complementarity determining regions 1-3 (CDRH1-3) and light chain complementarity determining regions 1-3 (CDRL1-3) having the following amino acid sequences:
(i) CDRH1: SEQ ID NO: 7 or a variant thereof having up to 1 or 2 amino acid substitutions compared with the sequence of SEQ ID NO: 7;
(ii) CDRH2: SEQ ID NO: 8 or a variant thereof having up to 1 or 2 amino acid substitutions compared with the sequence of SEQ ID NO: 8;
(iii) CDRH3: SEQ ID NO: 9 or a variant thereof having up to 1 or 2 amino acid substitutions compared with the sequence of SEQ ID NO: 9;
(iv) CDRL1: SEQ ID NO: 10 or a variant thereof having up to 1 or 2 amino acid substitutions compared with the sequence of SEQ ID NO: 10;
(v) CDRL2: SEQ ID NO: 11 or a variant thereof having up to 1 or 2 amino acid substitutions compared with the sequence of SEQ ID NO: 11; and
(vi) CDRL3: SEQ ID NO: 12 or a variant thereof having up to 1 or 2 amino acid substitutions compared with the sequence of SEQ ID NO: 12; optionally
wherein the antibody comprises CDRH1-3 comprising the amino acid sequences of SEQ ID NOS: 7-9, respectively; and CDRL1-3 comprising the amino acid sequences of SEQ ID NOS: 10-12, respectively.

13. The conjugate according to claims 11 or 12, wherein the antibody comprises:
a) a heavy chain variable region (VH) comprising an amino acid sequence having at least 90%, 95% or 99% sequence identity with the full-length sequence of SEQ ID NO: 5; optionally wherein the antibody comprises a heavy chain variable region (VH) comprising the amino acid sequence of SEQ ID NO: 5; and/or
b) a light chain variable region (VL) comprising an amino acid sequence having at least 90%, 95% or 99% sequence identity with the full-length sequence of SEQ ID NO: 6; optionally wherein the antibody comprises a light chain variable region (VL) comprising the amino acid sequence of SEQ ID NO: 6.

14. The conjugate according to any one of claims 11 to 13, wherein the antibody comprises:
a) a heavy chain comprising an amino acid sequence having at least 90%, 95% or 99% sequence identity with the full-length sequence of SEQ ID NO: 3; optionally wherein the antibody comprises a heavy chain comprising the amino acid sequence of SEQ ID NO: 3; and/or
b) a light chain comprising an amino acid sequence having at least 90%, 95% or 99% sequence identity with the full-length sequence of SEQ ID NO: 4; optionally wherein the antibody may comprise a light chain comprising the amino acid sequence of SEQ ID NO: 4.

15. The conjugate according to any one of claims 1 to 14, for use in a method of treating a disease in a mammalian subject.

16. The conjugate according to any one of claims 1 to 14, for use in a method of treating cancer in a mammalian subject; optionally
wherein the cancer comprises a solid tumour or a blood cancer, optionally wherein the cancer comprises gastric cancer, pancreatic cancer, Ewing sarcoma, breast cancer, melanoma, lung cancer, head & neck cancer, ovarian cancer, bladder cancer or colon cancer; further optionally wherein the cancer is an Endoglin-positive cancer,
further optionally wherein the conjugate is for simultaneous, sequential or separate administration with one or more other antitumour drugs, optionally wherein the one or more anti-tumour drugs comprises a cytotoxic chemotherapeutic agent or an anti-angiogenic agent or an immunotherapeutic agent, further optionally wherein the one or more anti-tumour drugs comprises Gemcitabine, Nab-paclitaxel, bevacizumab, itraconazole, carboxyamidotriazole, an anti-PD-1 molecule (for example, nivolumab, tislelizumab, or pembrolizumab) or an anti-PD-L1 molecule.

17. The conjugate for use according to claim 16, wherein the cancer comprises Endoglin-positive gastric cancer, and wherein the method comprises administering the conjugate intravenously at a dose of at least 5 mg/kg, 10 mg/kg, 15 mg/kg or at least 20 mg/kg.
